# EUROPEAN PATENT APPLICATION

(11) **EP 1 227 324 A2**
(43) Date of publication of application: **31.07.2002**
(21) Application number: 02250612.5
(22) Date of filing: 29.01.2002
(51) Int. Cl.: G01N 33/68, C12Q 1/48, C12Q 1/68, C12N 15/74, C12N 15/75, C12N 1/21, C07K 14/32, C07K 14/315, A61K 39/07, A61K 39/09, A61K 48/00

(54) **Har A polypeptides and nucleic acids and related methods and uses thereof**

(30) Priority: 30.01.2001 US 265034 P
(71) Applicant: Pfizer Products Inc., Groton, Connecticut 06340 (US)
(72) Inventor: Baima, Eric Todd, c/o Pfizer Global Res and Dev, Groton, Connecticut 06340 (US); Mueller, John Patrick Jr., c/o Pfizer Global R & D, Groton, Connecticut 06340 (US)
(74) Representative: Edwards, Fiona Anne

(57) **Abstract**

The present invention relates to harA polypeptides and nucleic acids encoding harA polypeptides and mutants thereof, derived from *Enterococcus faecalis* and *Bacillus subtilis,* as well as methods of using the polypeptides and nucleic acids in screening and diagnostic methods.

SEQUENCE LISTING

<110> Mueller, John P.
      Baima, Eric T.

<120> HAR A NUCLEIC ACIDS, POLYPEPTIDES, AND RELATED METHODS
      AND USES THEREOF

<130> PC11013

<140>
<141>

<160> 10

<170> PatentIn Ver. 2.1

<210> 1
<211> 1497
<212> DNA
<213> Enterococcus faecalis

<400> 1
   atgtcgaaaa ttgaactaaa acaactatct tttgcctatg ataatcaaga agtattgctt 60
   tttgatcagg caaatatcac gatggatacc aattggaaat taggattgat tggccgcaat 120
   ggccgtggga aaacaacctt attaagattg ttacaaaaac agttggatta ccaaggagag 180
   attcttcatc aagtcgattt cgtctatttt ccacaaacag ttgcagaaga acaacagctc 240
   acttattatg tcttacaaga ggtgacttct tttgaacagt gggaattaga acgagaatta 300
   acgcttttaa acgttgatcc tgaagtttta tggcggccct tttcttcttt atcaggcggc 360
   gaaaagacga aagttttatt aggtcttctt tttattgaag aaaatgcctt tcctttaatt 420
   gacgagccaa caaatcattt agatctagct ggcagacaac aagtggctga atatttgaag 480
   aaaaagaaac acgggtttat tttagtcagc cacgatcggg catttgttga tgaagtggtt 540
   gatcatattt tggcgattga aaaaagtcaa ttgacgctgt atcaagggaa tttttctatt 600
   tatgaagagc aaaaaaaatt aagagatgct tttgaactag cagaaaatga aaaaatcaaa 660
   aaagaagtca atcgcttgaa agaaaccgct cgtaaaaaag cggaatggtc gatgaaccgt 720
   gaaggtgata agtacggcaa cgctaaggaa aaagggagcg gggcgatttt tgatacagga 780
   gccattggtg cccgggcagc gcgcgtaatg aagcgctcga aacacattca acaacgcgcc 840
   gaaacacaat tagcagaaaa agaaaaacta ttaaaagatc ttgagtatat tgatcctttg 900
   tcaatggatt atcagccaac gcatcacaaa acattattga cggtggaaga gcttcgtcta 960
   ggctacgaga aaaattggct atttgcgcca ctttcttttt caataaacgc gggagaaatt 1020
   gttggaataa cagggaaaaa tggctcagga aaatcgagct taattcagta tttattggat 1080
   aatttttctg gggattcaga aggcgaagcc actttggctc accaattaac catttcttat 1140
   gtgcgccaag attatgaaga caatcaagga actttatccg aatttgcaga gaaaaatcag 1200
   ttagattaca ctcaattttt aaataactta cgaaaacttg ggatggagcg cgccgttttc 1260
   actaatcgaa ttgaacaaat gagtatgggg caacggaaaa aagtcgaagt agccaaatca 1320
   ttgtctcaat cagctgaact ttatatttgg gatgaacccc ttaattactt ggatgtattt 1380
   aatcatcaac aattagaagc gctaatctta tctgtgaagc ctgcaatgct agtgattgag 1440
   catgatgcac atttcatgaa gaaaataaca gataaaaaaa ttgtcttgaa atcataa    1497

<210> 2
<211> 498
<212> PRT
<213> Enterococcus faecalis

<400> 2
Met Ser Lys Ile Glu Leu Lys Gln Leu Ser Phe Ala Tyr Asp Asn Gln
  1               5                  10                  15

Glu Val Leu Leu Phe Asp Gln Ala Asn Ile Thr Met Asp Thr Asn Trp
             20                  25                  30

Lys Leu Gly Leu Ile Gly Arg Asn Gly Arg Gly Lys Thr Thr Leu Leu
        35                  40                  45

Arg Leu Leu Gln Lys Gln Leu Asp Tyr Gln Gly Glu Ile Leu His Gln
    50                  55                  60

Val Asp Phe Val Tyr Phe Pro Gln Thr Val Ala Glu Glu Gln Gln Leu
65                  70                  75                  80

Thr Tyr Tyr Val Leu Gln Glu Val Thr Ser Phe Glu Gln Trp Glu Leu
                85                  90                  95

Glu Arg Glu Leu Thr Leu Leu Asn Val Asp Pro Glu Val Leu Trp Arg
            100                 105                 110

Pro Phe Ser Ser Leu Ser Gly Gly Glu Lys Thr Lys Val Leu Leu Gly
        115                 120                 125

Leu Leu Phe Ile Glu Glu Asn Ala Phe Pro Leu Ile Asp Glu Pro Thr
    130                 135                 140

Asn His Leu Asp Leu Ala Gly Arg Gln Gln Val Ala Glu Tyr Leu Lys
145                 150                 155                 160

Lys Lys Lys His Gly Phe Ile Leu Val Ser His Asp Arg Ala Phe Val
                165                 170                 175

Asp Glu Val Val Asp His Ile Leu Ala Ile Glu Lys Ser Gln Leu Thr
            180                 185                 190

Leu Tyr Gln Gly Asn Phe Ser Ile Tyr Glu Glu Gln Lys Lys Leu Arg
        195                 200                 205

Asp Ala Phe Glu Leu Ala Glu Asn Glu Lys Ile Lys Lys Glu Val Asn
    210                 215                 220

Arg Leu Lys Glu Thr Ala Arg Lys Lys Ala Glu Trp Ser Met Asn Arg
225                 230                 235                 240

Glu Gly Asp Lys Tyr Gly Asn Ala Lys Glu Lys Gly Ser Gly Ala Ile
                245                 250                 255

Phe Asp Thr Gly Ala Ile Gly Ala Arg Ala Ala Arg Val Met Lys Arg
            260                 265                 270

Ser Lys His Ile Gln Gln Arg Ala Glu Thr Gln Leu Ala Glu Lys Glu
        275                 280                 285

Lys Leu Leu Lys Asp Leu Glu Tyr Ile Asp Pro Leu Ser Met Asp Tyr
    290                 295                 300

Gln Pro Thr His His Lys Thr Leu Leu Thr Val Glu Glu Leu Arg Leu
305                 310                 315                 320

Gly Tyr Glu Lys Asn Trp Leu Phe Ala Pro Leu Ser Phe Ser Ile Asn
                325                 330                 335

Ala Gly Glu Ile Val Gly Ile Thr Gly Lys Asn Gly Ser Gly Lys Ser
            340                 345                 350

Ser Leu Ile Gln Tyr Leu Leu Asp Asn Phe Ser Gly Asp Ser Glu Gly
        355                 360                 365

Glu Ala Thr Leu Ala His Gln Leu Thr Ile Ser Tyr Val Arg Gln Asp
    370                 375                 380

Tyr Glu Asp Asn Gln Gly Thr Leu Ser Glu Phe Ala Glu Lys Asn Gln
385                 390                 395                 400

Leu Asp Tyr Thr Gln Phe Leu Asn Asn Leu Arg Lys Leu Gly Met Glu
                405                 410                 415

Arg Ala Val Phe Thr Asn Arg Ile Glu Gln Met Ser Met Gly Gln Arg
            420                 425                 430

Lys Lys Val Glu Val Ala Lys Ser Leu Ser Gln Ser Ala Glu Leu Tyr
        435                 440                 445

Ile Trp Asp Glu Pro Leu Asn Tyr Leu Asp Val Phe Asn His Gln Gln
    450                 455                 460

Leu Glu Ala Leu Ile Leu Ser Val Lys Pro Ala Met Leu Val Ile Glu
465                 470                 475                 480

His Asp Ala His Phe Met Lys Lys Ile Thr Asp Lys Lys Ile Val Leu
                485                 490                 495

Lys Ser

<210> 3
<211> 1644
<212> DNA
<213> Bacillus subtilis

<400> 3
atgaaagaga tcgtaacatt aacaaacgtt agctatgaag taaaggatca aactgttttt 60
aaacatgtaa acgccagtgt tcagcaagga gatatcattg ggattatcgg caaaaacggc 120
gctgggaaat ctacgttgct gcacctcatt cacaatgact tagcccctgc acagggtcaa 180
atccttcgga aggatataaa actggctttg gttgaacagg aaaccgcggc gtattccttt 240
gcggatcaga cacctgccga aaagaagtta ctggagaaat ggcatgtgcc tcttcgtgat 300
tttcatcagt taagcggcgg tgaaaaactg aaagcgcggc tggcgaaagg actatcagag 360
gatgcagatc tgctgctgtt agatgaaccg acaaaccacc ttgatgaaaa aagcttgcaa 420
tttctcatcc aacagctgaa acattataac ggcactgtga ttctcgtttc tcacgatcga 480
tattttttag acgaagccgc aacaaaaata tggtcgcttg aggatcagac gctgattgaa 540
ttcaaaggga attactccgg gtatatgaag ttccgggaga agaaaagact cacccagcag 600
cgtgaatatg aaaagcagca aaaaatggtt gaacggattg aagcacaaat gaatgggctc 660
gcttcttggt cggaaaaagc ccatgctcaa tcgacgaaaa aggaagggtt taaagaatat 720
caccgggtaa aagcgaagcg tacggatgcc cagataaaat ccaagcagaa gcggcttgaa 780
aaagagcttg aaaaagcaaa ggcggaaccc gttaccccag aatatacagt ccgcttttca 840
atcgatacaa cccacaaaac aggaaaacgt tttttagaag ttcagaatgt aacaaaagcg 900
tttggagaaa ggactctctt taaaaacgca aactttacaa ttcagcacgg cgaaaaggtt 960
gcgatcatag gccccaatgg cagcggaaaa acgacattac tgaacatcat tctgggacag 1020
gaaacagcag aaggaagtgt atgggtgtcg ccgtccgcaa acatcggcta tttaacgcag 1080
gaggtgtttg atttgccttt agaacaaaca ccggaagagt tatttgagaa tgaaacattc 1140
aaagcaaggg ggcacgttca aaatctgatg aggcacttag gttttacagc cgcccaatgg 1200
actgaaccga tcaagcatat gagtatgggt gagcgtgtaa agatcaagct gatggcatat 1260
attctggagg aaaaagacgt gctgatttta gatgagccga caaaccatct cgacctgccg 1320
tcacgcgaac agctggaaga aacactgtca caatacagcg gcacattgct ggcggtttca 1380
catgaccgat actttctcga aaaaacaaca aacagtaaac tcgtcatctc aaacaacggc 1440

atcgaaaagc agttaaacga cgttccttca gaaagaaatg agcgggagga gcttcggtta 1500
aagcttgaga cagaaagaca agaagtgctg ggaaagctca gttttatgac gccaaatgat 1560
aaagggtata aggagcttga tcaggctttc aatgagctta cgaaacgaat aaaagagctg 1620
gatcatcaag acaaaaaaga ctga 1644

<210> 4
<211> 547
<212> PRT
<213> Bacillus subtilis

<400> 4
Met Lys Glu Ile Val Thr Leu Thr Asn Val Ser Tyr Glu Val Lys Asp
1               5                   10                  15

Gln Thr Val Phe Lys His Val Asn Ala Ser Val Gln Gln Gly Asp Ile
            20                  25                  30

Ile Gly Ile Ile Gly Lys Asn Gly Ala Gly Lys Ser Thr Leu Leu His
        35                  40                  45

Leu Ile His Asn Asp Leu Ala Pro Ala Gln Gly Gln Ile Leu Arg Lys
    50                  55                  60

Asp Ile Lys Leu Ala Leu Val Glu Gln Glu Thr Ala Ala Tyr Ser Phe
65                  70                  75                  80

Ala Asp Gln Thr Pro Ala Glu Lys Lys Leu Leu Glu Lys Trp His Val
                85                  90                  95

Pro Leu Arg Asp Phe His Gln Leu Ser Gly Gly Glu Lys Leu Lys Ala
            100                 105                 110

Arg Leu Ala Lys Gly Leu Ser Glu Asp Ala Asp Leu Leu Leu Leu Asp
        115                 120                 125

Glu Pro Thr Asn His Leu Asp Glu Lys Ser Leu Gln Phe Leu Ile Gln
    130                 135                 140

Gln Leu Lys His Tyr Asn Gly Thr Val Ile Leu Val Ser His Asp Arg
145                 150                 155                 160

Tyr Phe Leu Asp Glu Ala Ala Thr Lys Ile Trp Ser Leu Glu Asp Gln
                165                 170                 175

Thr Leu Ile Glu Phe Lys Gly Asn Tyr Ser Gly Tyr Met Lys Phe Arg
            180                 185                 190

Glu Lys Lys Arg Leu Thr Gln Gln Arg Glu Tyr Glu Lys Gln Gln Lys
        195                 200                 205

Met Val Glu Arg Ile Glu Ala Gln Met Asn Gly Leu Ala Ser Trp Ser
    210                 215                 220

Glu Lys Ala His Ala Gln Ser Thr Lys Lys Glu Gly Phe Lys Glu Tyr
225                 230                 235                 240

His Arg Val Lys Ala Lys Arg Thr Asp Ala Gln Ile Lys Ser Lys Gln
                245                 250                 255

Lys Arg Leu Glu Lys Glu Leu Glu Lys Ala Lys Ala Glu Pro Val Thr

260                 265                 270

Pro Glu Tyr Thr Val Arg Phe Ser Ile Asp Thr Thr His Lys Thr Gly
        275                 280                 285

Lys Arg Phe Leu Glu Val Gln Asn Val Thr Lys Ala Phe Gly Glu Arg
    290                 295                 300

Thr Leu Phe Lys Asn Ala Asn Phe Thr Ile Gln His Gly Glu Lys Val
305                 310                 315                 320

Ala Ile Ile Gly Pro Asn Gly Ser Gly Lys Thr Thr Leu Leu Asn Ile
                325                 330                 335

Ile Leu Gly Gln Glu Thr Ala Glu Gly Ser Val Trp Val Ser Pro Ser
            340                 345                 350

Ala Asn Ile Gly Tyr Leu Thr Gln Glu Val Phe Asp Leu Pro Leu Glu
        355                 360                 365

Gln Thr Pro Glu Glu Leu Phe Glu Asn Glu Thr Phe Lys Ala Arg Gly
    370                 375                 380

His Val Gln Asn Leu Met Arg His Leu Gly Phe Thr Ala Ala Gln Trp
385                 390                 395                 400

Thr Glu Pro Ile Lys His Met Ser Met Gly Glu Arg Val Lys Ile Lys
                405                 410                 415

Leu Met Ala Tyr Ile Leu Glu Glu Lys Asp Val Leu Ile Leu Asp Glu
            420                 425                 430

Pro Thr Asn His Leu Asp Leu Pro Ser Arg Glu Gln Leu Glu Glu Thr
        435                 440                 445

Leu Ser Gln Tyr Ser Gly Thr Leu Leu Ala Val Ser His Asp Arg Tyr
    450                 455                 460

Phe Leu Glu Lys Thr Thr Asn Ser Lys Leu Val Ile Ser Asn Asn Gly
465                 470                 475                 480

Ile Glu Lys Gln Leu Asn Asp Val Pro Ser Glu Arg Asn Glu Arg Glu
                485                 490                 495

Glu Leu Arg Leu Lys Leu Glu Thr Glu Arg Gln Glu Val Leu Gly Lys
            500                 505                 510

Leu Ser Phe Met Thr Pro Asn Asp Lys Gly Tyr Lys Glu Leu Asp Gln
        515                 520                 525

Ala Phe Asn Glu Leu Thr Lys Arg Ile Lys Glu Leu Asp His Gln Asp
    530                 535                 540

Lys Lys Asp
545

<210> 5
<211> 22
<212> DNA
<213> Enterococcus faecalis

<400> 5
tctagatatc acgatggata cc                                                22

<210> 6
<211> 23
<212> DNA
<213> Enterococcus faecalis

<400> 6
tctagattgc cgtacttatc acc                                               23

<210> 7
<211> 31
<212> DNA
<213> Bacillus subtilis

<400> 7
catatgaaag agatcgtaac attaacaaac g                                      31

<210> 8
<211> 31
<212> DNA
<213> Bacillus subtilis

<400> 8
ggatccttag tcttttttgt cttgatgatc c                                       31

<210> 9
<211> 19
<212> DNA
<213> Bacillus subtilis

<400> 9
catatgtcga aaattgaac                                                    19

<210> 10
<211> 21
<212> DNA
<213> Bacillus subtilis

<400> 10
ggatccttat gatttcaaga c                                                 21

## Description

### Field Of The Invention

The present invention relates to harA polypeptides and nucleic acids encoding harA polypeptides and mutants thereof, derived from *Enterococcus faecalis* and *Bacillus subtilis,* as well as methods of using the polypeptides and nucleic acids in compound screening and diagnostic methods.

### BACKGROUND OF THE INVENTION

The occurrence of bacterial resistance to commonly used antibiotic agents, often including resistance to multiple-agent regimens, is a growing problem worldwide. Many bacterial organisms exhibit resistance to a number of natural product (and related) antibacterial agents, either intrinsically, presumably due to the expression of endogenous gene products conferring such resistance, or as resistance acquired from other already resistant organisms. Among organisms known to be resistant to some antibacterial agents is *Enterococcus faecalis* ("E. faecalis"). E. faecalis infections are among the most common nosocomial (hospital-acquired) and iatrogenic (arising from treatment, e.g., needle puncture) infections, which in addition to compromising recovery can lead to sepsis, septic shock or even death. E. faecalis is also responsible for a significant fraction of prostatitis cases as well as urinary tract, pelvic, abdominal and neonatal infections, and is frequently the causative agent in bacterial endocarditis.

It is understood that the intrinsic level of bacterial susceptibility to antibacterial agents depends on both the ability of the agent to accumulate within the cellular compartment and the affinity of the agent for its specific target. Many drug-resistant bacteria express proteins which transport antibiotic drugs out of the bacterium back into the extracellular medium, preventing accumulation of the drug and reducing the susceptibility of the bacterium to the drug. These drug transport proteins may be divided into two classes: secondary transporters, which couple extrusion of drugs with ion exchange, and ATP-binding cassette ("ABC") transporters, which utilize the energy of a phosphate bond upon ATP hydrolysis to extrude the drug. While some transporters are very specific, recognizing only one drug class, multidrug transporters also exist, which extrude a wide variety of drug classes as well as other molecules such as detergents and dyes. See, Young, J., and Holland, I.B. "ABC transporters: bacterial exporters-revisited five years on," BBA 1461:177-200 (1999). Other mechanisms of resistance include drug inactivation, e.g., by enzymes at or within the cell surface, and target alteration, e.g., due to natural variation in ribosomal structures, or to the absence of a transport system required for entry of a drug into a cell.

Attempts have been made to analyze existing genomic resources to understand ABC-containing proteins, e.g., see, Quentin, Y, Fichant, G. and Denizot, F. "Inventory, Assembly and Analysis of Bacillus subtilis ABC Transport Systems" J. Mol. Biol 287:467-484 (1999). Quentin et al. performed an analysis of the B. subtilis genome and identified 78 ABC-containing genes, eight of which had not been annotated previously as ATP-binding proteins. In their scheme, transporters are characterized by the genomic association of three subunits - a nucleotide binding domain, a membrane spanning domain and solute binding protein - expressed as one or more polypeptides. However, one group of five ATPases in the "exporter" group (designated "subfamily 3") was not associated in the genome with any membrane-spanning domains, but exhibited some sequence similarity to proteins associated with macrolide resistance or with regulation of protein translation. The function of the genes in subfamily 3, one of which is the gene "expZ" described by applicants herein, has not been identified to date.

ABC sequences in other organisms can be identified in publicly accessible genetic databases such as Genbank, e.g., the genes designated YER036c, YFR009w, YPL226w, YLR249w, YNL014w, YDR091c, YKL209c, YLL015w, YOL075c, YCR011c and YHL035c. See, Linton, K.J. and Higgins, C.F., "The Escherichia coli ATP-binding cassette (ABC) proteins," Molecular Microbiology 28(1):5-13 (1998). A useful database of information relating to ABC proteins is available at http://ir2Icb.cnrs-mrs.fr/ABCdb/. The E. faecalis genome has now been sequenced, and many sequences are annotated with respect to their actual or possible functions. A publicly available site for analysis of E. faecalis genomics is http://pedant.mips.biochem.mpg.de.

While ABC-containing sequences can be identified within the growing genomic resources based on sequence characteristics common among ATP binding regions, it is not known how to identify which ABC-containing genes confer drug resistance, nor how to identify which drugs will be ineffective against an organism expressing a particular ABC-containing gene. One way to address the growing problem of antibiotic resistance, which applicants have pursued, is to attempt to identify transporters and their substrate antibacterial compounds, so as to identify genes whose expression may lead to antibiotic resistance in an organism. An example of efforts by others may be found in PCT International patent application WO 00/36101, which discloses an ABC protein called "MXR1" and states that overexpression of the protein confers resistance to the cytotoxic effects of several chemotherapeutic agents such as mitoxantrone. Another effort is disclosed in PCT International patent application WO 99/31133, which discloses nucleic acids encoding a Staphylococcus aureus polypeptide having homology to an *Archaeoglobus fulgidus* ABC transporter, but does not identify any compounds which are substrates for the encoded polypeptide.

### SUMMARY OF THE INVENTION

Applicants have discovered strains of *E. faecalis* and *B. subtilis* which are resistant to hygromycin A analogues, and identified a previously uncharacterized endogenous gene product which confers that resistance, as well as resistance to other antibacterial agents. The term "harA" (i.e., hygromycin A resistance) is used generally herein to refer to any polypeptide, or nucleic acid encoding such polypeptide, or a fragment or derivative of such polypeptide or nucleic acid, which confers hygromycin A resistance.

The present invention relates to harA polypeptides and nucleic acids encoding harA polypeptides derived from *E. faecalis* or *B. subtilis* and mutants thereof, as well as methods of producing harA polypeptides and harA nucleic acids, and compositions comprising the polypeptides and nucleic acids. The present invention also relates to uses for harA polypeptides and harA nucleic acids in diagnostic and vaccination methods, as well as methods and processes of using harA polypeptides and harA nucleic acids to identify compounds which bind to, modulate or inhibit the activity of the harA polypeptide, including the ATPase activity of the harA polypeptide.

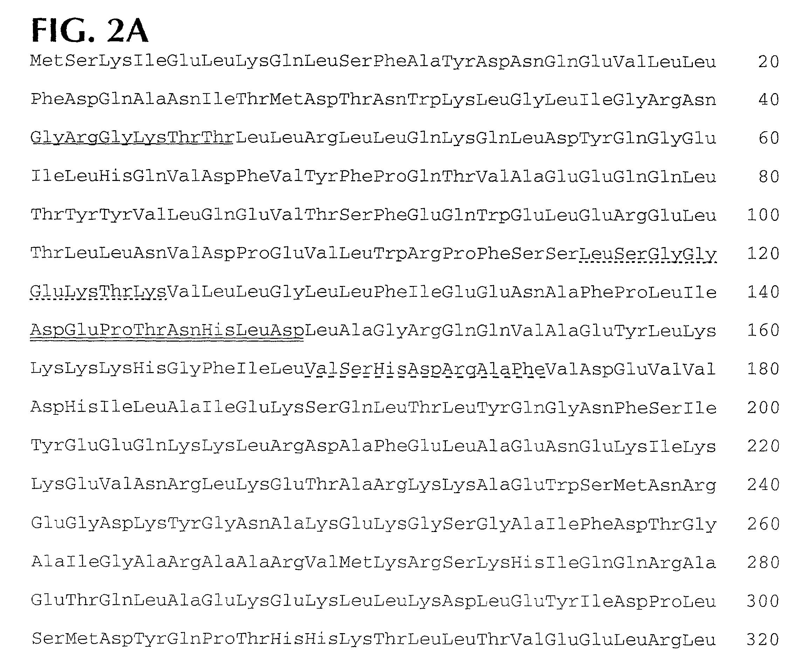

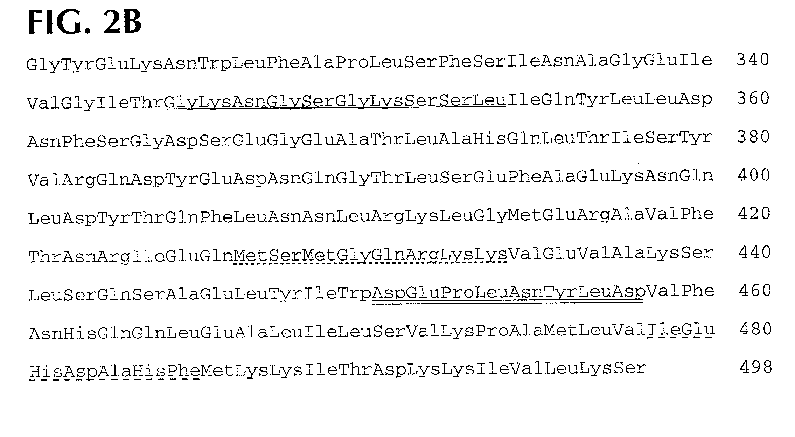

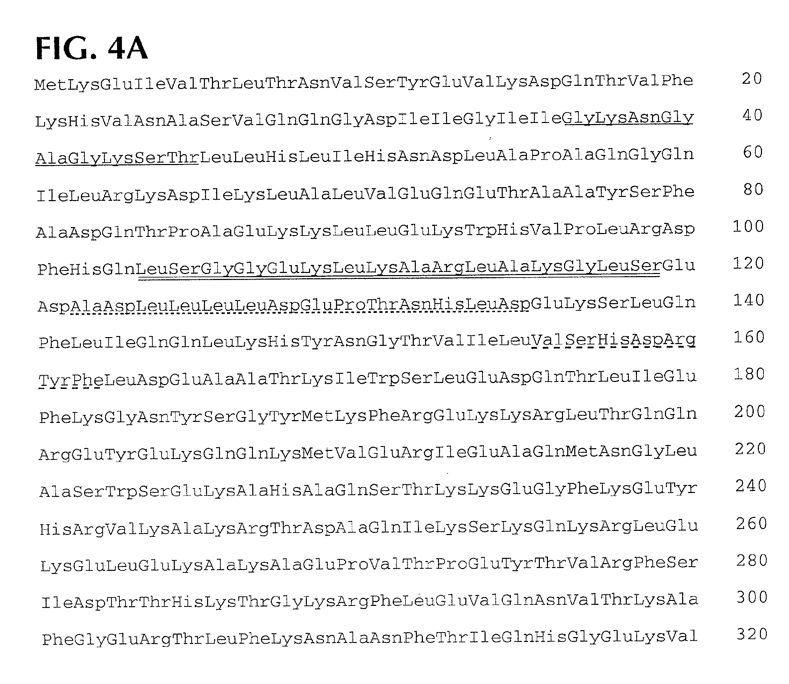

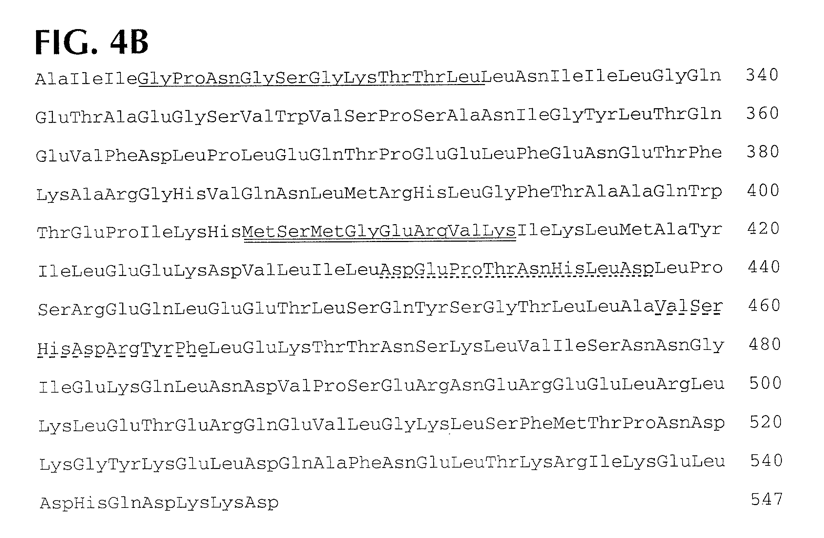

This invention relates to an isolated harA polypeptide comprising an amino acid sequence at least 75% identical to the amino acid sequence shown in Figure 2 (SEQ ID NO:2). In an embodiment, the polypeptide is an E. faecalis harA polypeptide. In another embodiment, the polypeptide is in substantially pure form. In another embodiment, the polypeptide comprises the amino acid sequence shown in Figure 2 (SEQ ID NO:2). The invention also provides an isolated harA polypeptide consisting essentially of the amino acid sequence shown in Figure 2 (SEQ ID NO:2). This invention also relates to an isolated harA polypeptide comprising an amino acid sequence at least 75% identical to the amino acid sequence shown in Figure 4 (SEQ ID NO:4). In an embodiment, the polypeptide is a Bacillus subtilis harA polypeptide. In another embodiment, the polypeptide is in substantially pure form. In another embodiment, the polypeptide comprises the amino acid sequence shown in Figure 4 (SEQ ID NO:4). The invention also provides an isolated harA polypeptide consisting essentially of the amino acid sequence shown in Figure 4 (SEQ ID NO:4). The invention also relates to a harA polypeptide having an amino acid sequence consisting essentially of the amino acid sequence encoded by the nucleic acid contained in the plasmid pGEM-T/harA (ATCC Accession No. PTA-2552). The invention also relates to a harA polypeptide having an amino acid sequence consisting essentially of the amino acid sequence encoded by the nucleic acid contained in the plasmid pMP-bacA1-1 (ATCC Accession No. PTA-2551).

The term "isolated" means altered by the hand of man from its natural state, i.e., if it occurs in nature, it has been changed or removed, or both, from its naturally occurring state. For example, a nucleic acid or polypeptide naturally present in a prokaryotic or eukaryotic cell, bacterium, virion or virus is not "isolated" but the same nucleic acid or polypeptide separated from the coexisting materials of its natural state is "isolated" as used herein. Also included in the meaning of the term "isolated" are e.g., any recombinant nucleic acid, cloned gene, in vitro transcription or translation product, exogenous or heterologous nucleic acid or its transcription and translation products and any nucleic acid or polypeptide processed by chemical, enzymatic or biotransformative means including radiolabeling and substitution of modified or otherwise non-naturally occurring nucleic or amino acids.

"Polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. "Polypeptide(s)" include those modified either by natural processes, such as processing and other post-translational modifications, but also by chemical modification techniques. Such modifications are described in basic texts, in more detailed monographs, and research publications and are well known in the art. It is to be understood that the same type of modification may be present in the same or varying degree at several sites in a given polypeptide. Also, a given polypeptide may contain many types of modifications. Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation (e.g., formation of hydroxyproline or hydroxylysine), iodination, methylation (e.g., formation of e-N-methyllysine, 3-methyl-histidine), myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, PROTEINS--STRUCTURE AND MOLECULAR PROPERTIES, 2^{nd} Ed., T. E. Creighton, W. H. Freeman and Company, New York (1993) and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York (1983); Seifter et al., Meth. Enzymol. 182:626-646 (1990) and Rattan et al., Protein Synthesis: Posttranslational Modifications and Aging, Ann. N.Y. Acad. Sci. 663: 48-62 (1992). Polypeptides may be branched or cyclic with or without branching. Cyclic, branched and branched circular polypeptides may result from post-translational natural processes and may be made by entirely synthetic methods, as well. Polypeptides may contain amino acids other than the 20 naturally-encoded amino acids, e.g., β-, γ-, or δ-amino acids, or D-amino acids, which are well-known in the art.

In this application, the meaning of "isolated polypeptide" also includes any preparation comprising native or exogenous lipids, including detergents, membrane preparations from cells expressing a harA polypeptide, as well as vesicular or closed membranous preparations comprising a harA polypeptide.

"Identical to" or "identity" as used interchangeably in this application, is a relationship between two or more polypeptide sequences or two or more polynucleotide sequences, as determined by comparing the sequences. In this application, "similar to" or "similarity" means the degree of sequence relatedness between polypeptide or polynucleotide sequences, as the case may be, as determined by the match between strings of such sequences. "Identity" and "similarity" can be readily calculated by known methods, including but not limited to those described in, e.g.: Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research 12(1): 387 (1984)), BLASTP, BLASTN, and FASTA (Altschul, S. F. et al., J. Molec. Biol. 215: 403-410 (1990). The BLAST X program is publicly available from NCBI and other sources (BLAST Manual, Altschul, S., et al., NCBI NLM NIH Bethesda, Md. 20894; Altschul, S., et al., J. Mol. Biol. 215: 403-410 (1990). The well known Smith-Waterman algorithm may also be used to determine identity.

Particularly preferred parameters for polypeptide sequence comparison include the following: Algorithm: Needleman and Wunsch, J. Mol Biol. 48: 443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "gap" program from Genetics Computer Group, Madison, Wis. The aforementioned parameters are the default parameters for peptide comparisons (along with no penalty for end gaps).

This invention also relates to a harA polypeptide comprising an amino acid sequence having at least 75, 80, 85, 90, 95, 97 or 100% identity to a polypeptide reference sequence. In an embodiment, the harA polypeptide may be identical to the reference sequence or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino-or carboxy-terminal positions of the reference polypeptide sequence or anywhere between those terminal positions, interspersed either individually among the amino acids in the reference sequence or in one or more contiguous groups within the reference sequence. In a preferred embodiment, the reference sequence is the amino acid sequence shown in SEQ ID NO: 2. In another preferred embodiment, the reference sequence is the amino acid sequence shown in SEQ ID NO: 4.

This invention also relates to a harA polypeptide comprising an amino acid sequence having conservative substitutions not exceeding 10% of the amino acids comprising a reference sequence. In an embodiment, the reference sequence is the amino acid sequence shown in SEQ ID NO:2. In another embodiment, the reference sequence is the amino acid sequence shown in SEQ ID NO:4. In a preferred embodiment, the harA polypeptide comprises conservative amino acid substitutions in any one or more of the ABC domains of the polypeptide. In this application, the term "ABC domain" comprises any sequence containing at least a Walker A box and at least a Walker B box. In a more preferred embodiment, one or more of the Walker A, Walker B, signature motif or downstream motif sequences, as identified in Figure 2 (SEQ ID NO:2), are conserved. In another more preferred embodiment, one or more of the Walker A, Walker B, signature motif or downstream motif sequences, as identified in Figure 4 (SEQ ID NO:4), are conserved. In another preferred embodiment, the harA polypeptide comprises conservative amino acid substitutions in the regions of a harA polypeptide outside any one or more of the ABC domains, Walker A, Walker B, signature motif or downstream motif sequences.

"Conservative" amino acid substitutions are well-known in the art. Rules for making such substitutions include those described by Dayhof, M.D., 1978, Nat. Biomed. Res. Found., Washington, D.C., Vol. 5, Sup. 3, among others. More specifically, conservative amino acid substitutions are those that generally take place within a family of amino acids that are related in acidity, polarity, or bulkiness of their side chains. Genetically encoded amino acids are generally divided into four groups: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cysteine, serine, threonine, and tyrosine. Phenylalanine, tryptophan and tyrosine are also jointly classified as aromatic amino acids. One or more replacements within any particular group, e.g., of a leucine with an isoleucine or valine, or of an aspartate with a glutamate, or of a threonine with a serine, or of any other amino acid residue with a structurally related amino acid residue, e.g., an amino acid residue with similar acidity, polarity, bulkiness of side chain, or with similarity in some combination thereof, will generally have an insignificant effect on the function or immunogenicity of the polypeptide. In a preferred embodiment, the conservatively substituted polypeptide has at least about 75%, more preferably at least about 85%, and even more preferably at least about 90% sequence identity, and most preferably at least 95% sequence identity to SEQ ID NO: 2 or SEQ ID NO:4.

In another embodiment, the polypeptide consists of a harA polypeptide having between 1 and 10, and more preferably between 1 and 5, amino acids inserted, deleted, or substituted, including combinations thereof. In a more preferred embodiment, the isolated polypeptide has between 1 and 5 amino acids conservatively substituted in the amino acid sequence of the harA polypeptide.

The present invention further relates to harA polypeptides consisting of a substantial portion of any one of the aforementioned polypeptides of the present invention. As used herein, a "substantial portion" of a polypeptide of the present invention, or "peptide fragment," means a polypeptide consisting of less than the complete amino acid sequence of the corresponding full-length polypeptide, but comprising at least about 75%, more preferably at least about 80%, even more preferably at least about 90%, and most preferably at least about 95% of the amino acid sequence thereof.

This invention relates to an isolated harA polypeptide in substantially pure form. In an embodiment, the isolated harA polypeptide is recovered in soluble form. In another embodiment, the isolated harA polypeptide is recovered in insoluble form.

In this application, "substantially pure" means comprising at least 90% by weight, preferably at least 95% by weight, and more preferably at least 99% by weight of a single substance, wherein a substance is any chemical compound, whether naturally occurring or non-naturally occurring.

The term "recovered in a soluble form" indicates that a protein or polypeptide is retrieved from the cytoplasm of the cell that expresses said protein or polypeptide. A protein or polypeptide recovered in an emulsion, detergent, or membranous preparation is in this invention also considered to be recovered in a soluble form.

The term "recovered in an insoluble form" indicates that a protein or polypeptide is retrieved from inclusion bodies present in the cell that expresses said protein or polypeptide.

The present invention further relates to a method of preparing any of the polypeptides described herein, which comprises culturing host cells transformed with any of the recombinant expression vectors described herein, and recovering the expressed polypeptide from the cell culture, in either a soluble or an insoluble form. Culturing is conducted under conditions conducive to expression of the polypeptide as described further herein and as particularly preferred according to the selected host cell, which conditions are well-known in the art.

This invention relates to an immunological composition comprising a harA polypeptide and a pharmaceutically acceptable carrier. This invention also relates to an immunological composition comprising a nucleic acid encoding a harA polypeptide, wherein the nucleic acid is operatively associated with a vector effective to direct expression of the polypeptide in an animal, and a pharmaceutically acceptable carrier.

In an embodiment of either of the above compositions, the carrier further comprises an adjuvant or other modulating agent effective to stimulate or enhance the immune response of the animal against the harA polypeptide. Adjuvants for use as immune stimulants and in vaccines are well-known in the art, e.g., Freund's adjuvant, Amphigen, and other commercially available adjuvants, and are adapted for use in a selected animal, mammal or human by reference to established clinical guidelines.

In other embodiments, the composition is in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. In a preferred embodiment thereof, the composition is a unit dosage form suitable for single administration of precise dosages.

This invention relates to a method of treating a drug-resistant infection in an animal which comprises administering to the animal any of the above compositions in an amount sufficient to elicit an increase in susceptibility to the drug. In an embodiment, the drug resistance is mediated by a harA polypeptide or by expression of a harA nucleic acid. In another embodiment, the drug is a hygromycin A or related analogue, a virginiamycin, a lankacidin or Synercid®. In this application, the term "analogue(s)" when applied to any chemical compound includes not only the named compound, but also compounds in the same chemical structural class and compounds derived therefrom. Accordingly, the use of the phrase "a hygromycin A" (or, similarly, "a lankacidin", "a virginiamycin" "a streptogramin"), refers to a compound or compounds in that chemical structural class. The term "hygromycin(s)" as used herein, means a hygromycin A. In an embodiment, the infection is a bacterial infection, and in a preferred embodiment thereof, the bacterial infection is an E. faecalis, Streptococcus pyogenes, Streptococcus pneumoniae, Haemophilus influenzae or Enterococcus faecium infection. In a more preferred embodiment thereof, the bacterial infection is an Enterococcus faecium or E. faecalis infection. In another more preferred embodiment, the bacterial infection is a Streptococcus pyogenes or Streptococcus pneumoniae infection. In another more preferred embodiment, the bacterial infection is a Haemophilus influenzae infection. In another embodiment, the infection is a protozoal infection. In a preferred embodiment thereof, the protozoal infection is a Serpulina (Treponema) hyodysenteriae infection. In another embodiment thereof, the infection is by any infectious agent identified as amenable to treatment with a hygromycin A. In another embodiment, the infection is any one of a hygromycin A-resistant, virginiamycin-resistant, lankacidin-resistant or Synercid®-resistant infection. In still another embodiment, the infection is resistant to a hygromycin A analogue. In another embodiment, the infection is vancomycin-resistant. In other embodiments, the method comprises administering to the animal any of the above compositions in combination with another antimicrobial compound or composition which treats, e.g., an Enterococcus, Streptococcus or Hemophilus infection. In an embodiment, the other antimicrobial compound is a macrolide (e.g., erythromycin, clarithromycin, azithromycin), a beta-lactam (e.g., penicillins, cephalosporins), an aminoglycoside, a sulfonamide, a tetracycline, a quinolone, a streptogramin an oxazolidinone or a glycopeptide (e.g., vancomycin). It is to be understood that the above examples are illustrative only and are not intended to be limiting as to the specific antimicrobial agents to be used in the present invention.

In an embodiment of any of the above compositions or methods, the animal is a mammal. In a more preferred embodiment thereof, the mammal is a human. In other more preferred embodiments, the mammal is a companion animal to humans, including e.g., canines or felines, or the mammal is a livestock animal, including e.g., an equine, bovine, or porcine animal. In other embodiments, the animal is an avian companion animal or a poultry animal.

In an embodiment of any of the above compositions or methods, the polypeptide is a harA polypeptide comprising an amino acid sequence at least 75% identical to the amino acid sequence shown in Figure 2 (SEQ ID NO:2). In another embodiment, the polypeptide is a harA polypeptide comprising an amino acid sequence at least 75% identical to the amino acid sequence shown in Figure 4 (SEQ ID NO:4). In another embodiment, the polypeptide is a harA polypeptide comprising the amino acid sequence shown in Figure 2 (SEQ ID NO:2), or the amino acid sequence shown in Figure 4 (SEQ ID NO:4). In another embodiment, the polypeptide is a fragment or variant of a harA polypeptide such as the polypeptides shown in Figure 2 (SEQ ID NO:2) or Figure 4 (SEQ ID NO:4). In a preferred embodiment of any of the above compositions or methods, the animal is a mammal. In a more preferred embodiment, the mammal is a human. In another more preferred embodiment, the mammal is selected from among porcine, bovine, equine, canine and feline mammals.

An "immunological composition" is one which is capable of eliciting an immune response in an animal, which response is directed specifically against a polypeptide or protein. The terms "immunogenic" and "immunogenicity" refer to the capability of a polypeptide to elicit an immune response directed specifically against the polypeptide. The terms "antigenic" and "antigenicity" refer to the capability of a protein or polypeptide to be specifically bound by an antibody to the protein or polypeptide.

The term "pharmaceutically acceptable carrier" refers to a substance that allows delivery and absorption of an active ingredient, such as a drug substance, polypeptide or nucleic acid, and does not interfere with the effectiveness of the biological activity of the active ingredient, is chemically inert and is not toxic to the subject to whom it is administered.

Suitable pharmaceutical carriers include inert diluents or fillers, water and various organic solvents. The pharmaceutically acceptable carrier may, if desirable, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefor, include lactose or milk sugar and high molecular weight polyethylene glycols. When aqueous suspensions or elixirs are desired for oral administration the carrier may contain various sweetening or flavoring agents, coloring matters or dyes and, if desired, emulsifying agents or suspending agents, together with diluents such as water, ethanol, propylene glycol, glycerin, aqueous propylene glycol or dextrose solutions or combinations thereof. The carrier and the active ingredient are preferably sterile. Dosage forms can be suitably buffered, if desired.

Methods of preparing various pharmaceutical compositions with a specific amount of active compound are known, or will be apparent, to those skilled in this art. For examples, see Remington's Pharmaceutical Sciences, Mack Publishing Company, Easter, Pa., 15th Edition (1975).

The term "treating or preventing a bacterial infection" means to inhibit the replication of the bacteria, to inhibit transmission of the infection, or to prevent the organism from establishing itself in its host, or to alleviate the symptoms of the disease caused by the infection. In preferred embodiments, the methods are used to treat an E. faecalis infection. In other preferred embodiments, the methods are used to treat one or more of a Streptococcus pyogenes, Streptococcus pneumoniae, Haemophilus influenzae or Enterococcus faecium infection. A treatment is considered therapeutically effective if there is a reduction in bacterial load, decrease in infections (i.e., reduced transmission) and/or increase in food uptake and/or growth.

This invention relates to an antibody which is immunoreactive against a harA polypeptide as described herein. In an embodiment, the antibody is a monoclonal antibody. In another embodiment, the antibody is a humanized antibody. In a preferred embodiment, the antibody is a humanized monoclonal antibody. In yet another embodiment, the antibody is immunoreactive against a polypeptide which is functionally equivalent to a harA polypeptide. The polypeptides and antibodies of the invention are useful for surveillance and detection of organisms resistant to hygromycin A analogues, virginiamycins, lankacidin and Synercid®.

This invention relates to a method for identifying the presence of harA-mediated antibiotic resistance in an animal, which comprises isolating from the animal any bacteria present therein, isolating the polypeptides expressed by the bacteria, and identifying the presence of a harA polypeptide by detecting binding of an antibody immunoreactive against a harA polypeptide to a protein isolated from the bacteria. In an embodiment, the animal is suffering from an antibiotic-resistant infection. In another embodiment, the animal is not suffering from an antibiotic-resistant infection. Accordingly, the antibodies of the present invention are useful both to direct the treatment of an infected animal, and for surveillance of an animal or population of animals which may become infected with an antibiotic resistant infection. In an embodiment, the antibiotic is a hygromycin A, a virginiamycin, a lankacidin, or Synercid®.

The term "antibody", as used herein, refers to an immunoglobulin molecule able to bind to an antigen. Antibodies can be a polyclonal mixture or monoclonal. Antibodies can be intact immunoglobulins derived from natural sources or from recombinant sources and can be immunoreactive portions of intact immunoglobulins. In an embodiment, the epitope comprises preferably more than 8, more preferably more than 12, and most preferably, more than 20 amino acids of the polypeptide sequence. The term "antibody" encompasses the full range of forms including, for example, Fv, Fab', F(ab')₂, as well as in single chains. Single chain antibodies, in which genes for a heavy chain and a light chain are combined into a single coding sequence, are also encompassed by the term "antibody".

The term "immunoreactive" as used in this application means capable of binding specifically to a polypeptide or protein.

The term "humanized" means the complementarity determining regions of the antibody has been transferred to a human monoclonal antibody, for example, as described in Jones, P. et al. Nature 321:52-525 (1986) or Tempest et al., Biotechnology 9:266-273 (1991). The term "humanized" also means an antibody comprising both human heavy chains and human light chains, such as the antibodies produced by transgenic mice engineered to express human antibodies against an arbitrary human or non-human antigen, for example as described in J. Immunol. Meth. 231:11-23 (1999).

The term "functionally equivalent", as utilized herein, refers to a protein capable of being recognized by an antibody specific to harA, that is a protein capable of eliciting a substantially similar immunological response as the wild-type harA protein. Thus, an antibody raised against a functionally equivalent protein will also recognize harA.

The term "protection" or "protecting", as used herein with respect to a vaccine, means that the vaccine prevents or reduces the symptoms of the disease caused by the organism from which the antigen(s) used in the vaccine is derived.

The term "effective amount" refers to an amount of harA nucleic acid or harA polypeptide sufficient to elicit an immune response in the subject to which it is administered. The immune response may comprise, without limitation, induction of cellular and/or humoral immunity.

The term "therapeutic agent" refers to any molecule, compound or treatment, preferably an antibacterial, that assists in the treatment of a bacterial infection or the diseases caused thereby.

"Variant(s)" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to skilled artisans.

The polypeptides and nucleic acids of this invention may be used to assess the binding of compounds to isolated harA polypeptides, e.g., harA polypeptides contained in, e.g., cells, cell-free preparations, membranous preparations or any other preparations suitable for binding and functional assays as described herein.

This invention relates to the use of a harA polypeptide to vaccinate an animal against infection by an organism which expresses a harA polypeptide.

This invention also relates to the use of a harA polypeptide to identify compounds which bind to a harA polypeptide. This invention also relates to the use of a harA polypeptide to identify compounds which inhibit the activity of a harA polypeptide. In an embodiment, the activity of a harA polypeptide is ATP hydrolysis, i.e., the harA polypeptide functions at least as an ATPase. Other activities are described further below.

This invention relates to a method of screening compounds to identify those which bind to a harA polypeptide. In this invention, a compound being screened to determine whether it binds to or affects the activity of a harA polypeptide, or interacts with or affects a cell expressing a harA polypeptide, may be referred to as a "candidate". In this invention, a plurality of compounds (or candidates) being screened to determine whether any of the compounds binds to or affects the activity of a harA polypeptide, or interacts with or affects a cell expressing a harA polypeptide, may be referred to as a "library of compounds". A compound, candidate, or library of compounds may be produced biosynthetically or by chemical synthetic methods, by biotransformation of naturally or synthetically produced compounds, or by a combination of such methods, as are well known in the art. The terms "bind(s)" or "binding" as used herein refer to "specific binding" as that phrase is understood in the art relating to a particular assay. Similarly, the "activity" of a polypeptide or nucleic acid means the activity which is dependent upon and results from the activity of the particular polypeptide or nucleic acid of interest, as opposed to constitutive activity or generation of a detectable signal by other cellular components. Specific examples of assays and controls therefor are described in more detail below and in the publications cited herein.

This invention relates to a method of screening a compound to determine whether the compound binds to a harA polypeptide, which comprises contacting the harA polypeptide with the compound under conditions suitable for binding, and detecting whether the compound binds to the harA polypeptide. In an embodiment, binding is detected directly, i.e., a detectable signal is incorporated into the compound. In an embodiment thereof, the signal is radiation emitted by one or more atoms of the compound which has been replaced by a radioisotope of the atom(s). In another embodiment, binding is detected by measurement of the harA polypeptide; in embodiments thereof, the harA polypeptide may be radiolabeled, or may comprise chemically modified amino acids or may comprise an antigenic sequence(s) of amino acids, or may comprise a fusion protein wherein the fusion partner may be used to detect harA polypeptide, e.g., the FLAG epitope. In another embodiment, binding is detected indirectly, i.e., a signal is generated by a molecule other than the compound or the harA polypeptide, when the compound binds to the harA polypeptide. In an embodiment thereof, the signal may be provided by the production of another polypeptide or by expression of a nucleic acid, e.g., as in a reporter gene system.

This invention relates to a method of screening a library to determine whether at least a compound from the library binds to a harA polypeptide, which comprises (a) contacting the harA polypeptide with the library under conditions suitable for binding, (b) detecting whether at least a compound from the library binds to the harA polypeptide, and if binding of at least a compound is detected, then (c) identifying each compound which binds to the harA polypeptide. It is to be understood that the compound identified may be a previously known compound or in the case of a library of compounds, may also include a compound not previously isolated but identifiable by its mode of synthesis, location or other indicium or indicia particular to the method by which the compound or library of compounds is made, or by isolation using well-known chemical techniques, either in association with or separate from the harA polypeptide or fragment thereof. This invention includes and encompasses high throughput screening methods, which are well known in the art. In an embodiment, step (b) and step (c) are performed together, i.e., the detection of binding of a compound from the library and identification of such compound are coupled or inseparable. In another embodiment, step (c), identification, is performed by isolating each compound in the library and separately determining for each such compound whether the compound binds to the harA polypeptide. Numerous configurations for identification of compounds using high throughput screening methods are well-known in the art, and this is not intended to be an exclusive listing thereof.

This invention relates to a method of screening a compound to determine whether the compound inhibits harA activity, which comprises: measuring the activity of a harA polypeptide (i) in the absence and (ii) in the presence of the compound, under conditions suitable for measuring harA activity; wherein a decrease in harA activity in the presence of the compound compared to the harA activity in the absence of the compound identifies the compound as inhibiting harA activity, wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4). In a preferred embodiment, as further described below, the harA activity measured is NTPase activity.

This invention relates to a method of screening compounds to identify compounds which inhibit the nucleotide triphosphatase ("NTPase") activity of a harA polypeptide. The invention relates to a method of screening a compound to determine whether the compound inhibits harA NTPase activity, which comprises measuring the NTPase activity of a harA polypeptide separately (i) in the absence and (ii) in the presence of the compound, under conditions suitable for measuring harA NTPase activity, and detecting a decrease of harA NTPase activity in the presence of the compound, compared to the harA NTPase activity in the absence of the compound. Methods of measuring NTPase activity are well known in the art. For example, NTPase activity may be measured as described in Hwang, K.Y., Chung, J.H., Kim, S.-H., Han, Y.S. and Cho, Y., "Structure-based identification of a novel NTPase from Methanococcus jannaschii," Nature Structural Biology 6(7):691-696 (1999). In an embodiment, the NTPase activity is ATPase activity, i.e., the nucleotide is adenosine. Methods of measuring ATPase activity are well known in the art. For example, ATPase activity may be measured as described in Zarembinski, T.I. et al., "Structure-based assignment of the biochemical function of a hypothetical protein: a test case of structural genomics," PNAS(USA) 95:15189-15193 (1998), or e.g., as described in Kiel, M.C., Hiroyuki, A., Ganoza, M.C., "Identification of a ribosomal ATPase in Escherichia coli cells," Biochimie 81:1097-1108 (1999). All of the above publications are herein incorporated by reference. In another embodiment, the ATPase activity is measured using cells expressing a harA polypeptide. In an embodiment thereof, the cells comprise the plasmid pMP-bacA1-1 (ATCC Accession No. PTA-2551). In another embodiment thereof, the cells comprise the plasmid pGEM-T/harA (ATCC Accession No. PTA-2552). In another embodiment, the ATPase activity is measured using isolated harA polypeptide. In another embodiment, ATPase activity is indicated by ATP-dependent protein synthesis.

This invention also relates to a method of screening compounds involving assays for harA activities related to transcription or translation as are well known in the art. See, e.g., Zubay, G. Ann. Rev. Genet. 7:267-287 (1973); also Pratt, J.M., "coupled transcription-translation in prokaryotic cell-free systems, p. 179-209, *in* B.D. Hames and S.J. Higgins (ed.) Transcription and Translation, A Practical Approach IRL Press, Oxford (1984); and Shinabarger et al. "Mechanism of Action of Oxazolidinones: Effects of Linezolid and Eperezolid on Translation Reactions," Antimicrobial Agents and Chemotherapy 41(10):2132-2136 (1997), as well as Kiel et al. (supra) which also describes poly(U)-programmed polyphenylalanine synthesis, Murray et al., Antimicrobial Agents and Chemotherapy 42(4):947 (1998), Mahmood et al., Gene 106:29-34 (1991), and Mao, J.C.-H. J. Bacteriol. 94:80-86 (1967). Regarding ABC-containing proteins, see, e.g., Tyzack et al. who describe the elevation of ABC50 mRNA in synoviocytes in response to TNF-alpha stimulation, as well as increases in levels of translational machinery, e.g., elongation factor elF2 in T-lymphocytes, in response to stimulation. Tyzack, J.K., Wang, X., Belsham, G.J. and Proud, C.G. "ABC50 Interacts with Eukaryotic Initiation Factor 2 and Associates with the Ribosome in an ATP-dependent manner" J. Biol. Chem. 275(44):34131-34139 (2000). Applicants herein incorporate by reference all of the above publications. The invention relates to any of the above methods of screening a compound, or a library of compounds, wherein binding of the compound is detected by a change in mRNA levels of a harA nucleic acid, by a change in the association with ribosomes or polysomes of one or more translation factors, or by a change in the levels of any detectable initiation factor. In an embodiment, the change in mRNA levels of a harA nucleic acid are detected by an in vitro translation assay such as are well-known in the art as described above.

In an embodiment of any of the above methods of screening, the binding of a compound to a harA polypeptide, or the effect of a compound on the activity of a harA polypeptide, e.g., ATPase activity or its effects on translation, may be detected by using reporter system. In an embodiment, the reporter system is a reporter gene system. Reporter gene systems are well-known in the art, see, e.g., Schenborn E. and Groskreutz, D. "Reporter gene vectors and assays," Mol. Biotechnol. 13(1):29-44 (1999). In a preferred embodiment thereof, the reporter gene system is coupled to ATPase activity. In a more preferred embodiment, the reporter gene system is responsive to ATP hydrolysis. In another embodiment, binding of a compound or group of compounds is detected by ELISA. In an embodiment thereof, the ELISA assay utilizes an antibody specific to a harA polypeptide. In another embodiment, the harA polypeptide is in a preparation derived from cells expressing the harA polypeptide, e.g., a cellular compartment, cell envelope, cell wall or membranous preparation, or may be derived from an in vitro expression system producing a harA polypeptide. In other embodiments, the interaction of a compound with a harA polypeptide is measured by an increased amount of the compound in cells expressing the harA polypeptide, in comparison to the amount of the compound in cells lacking harA or expressing a harA polypeptide which is disrupted or non-functional, e.g., in further embodiments, such cells may be JH642 expZ::CAT (ATCC Accession No. PTA-2480) or OG1X harA::ERM (ATCC Accession No. PTA-2550). An example of a method to measure the amount of a compound may be found in e.g., Sutcliffe, J., Tait-Kamradt, A. And Wondrack, L., "Streptococcus pneumoniae and Streptococcus pyogenes Resistant to Macrolides but Sensitive to Clindamycin: a Common Resistance Pattern Mediated by an Efflux System," Antimicrobial Agents and Chemotherapy, 40(6):1817-1824 (1996).

The invention also relates to a method of screening compounds to identify a compound which decreases resistance of an organism to an antibacterial agent, e.g., a hygromycin A, a lankacidin, a virginiamycin, a streptogramin or streptogramin combination, e.g., Synercid, or any other antibacterial agent(s) described herein, which comprises contacting an organism, wherein the organism is resistant to the agent due to expression of a harA polypeptide, with the compound under conditions suitable for measuring resistance of the organism to the agent, and detecting whether the compound decreases the resistance of the organism to the agent.

In an embodiment, the organism is contacted with a library of compounds, and if a decrease in the resistance of the organism is detected, further comprises identifying each compound in the library which decreases the resistance of the organism to the agent. It is to be understood that the compound identified may be a previously known compound or in the case of a library of compounds, may also include a compound not previously isolated but identifiable by its mode of synthesis, location or other indicium or indicia particular to the method by which the compound or library of compounds is made, or by isolation using well-known chemical techniques, either in association or separate from the harA polypeptide or fragment thereof.

In an embodiment of any of the above screening methods, decreased resistance is measured by a decrease in the MIC of the agent against the organism, relative to the MIC in the absence of the compound. In a preferred embodiment thereof, the MIC is measured using a broth microdilution assay. In a preferred embodiment, the decrease in the MIC is at least four-fold. In a more preferred embodiment thereof, the decrease in the MIC is at least about eight-fold (8-fold). In still more preferred embodiments, the decrease in the MIC is at least about 16-fold, or at least about 32-fold, or at least about 50-fold, or at least about 100-fold, or at least about 500-fold. The MIC, or "minimum inhibitory concentration", as used in this application, is the lowest concentration of antibiotic that completely inhibits growth.

"Resistant" or "drug resistant" (or "resistance" or "drug-resistance", respectively) as used in this application means failure of a drug (interchangeably, "compound" or "agent') to kill a particular strain of an organism, and is not intended to imply any quantitative measurement unless specifically noted herein (see below). Resistance may be manifested as survival of a subpopulation(s) of an organism throughout repeated cell division cycles of the organism in the presence of the drug, i.e., wherein at least a majority of cells in the population are killed but some proportion (the subpopulation(s)) survive and increase in number with each cell division cycle. Resistance may also be manifested as an increase in the MIC of a drug compared to the MIC of the drug against the organism upon initial exposure of the organism to the drug or upon deliberate introduction into a susceptible strain of an organism a gene known to confer resistance to the drug in other organisms or strains thereof. Preferably, changes in the MIC are at least about 4-fold, or at least about 8-fold, or about 16-fold, or about 32-fold, or about 50-fold, or about 100-fold or about 500-fold. Resistance may also be manifested by the absence of an inhibition zone in a disk-diffusion assay using a particular antibacterial agent and a particular test organism. Resistance may be inherent in a particular organism, or strain thereof, and identified only by the increased survival of the organism or strain thereof, in screening experiments by comparison with other related organisms or strains thereof.

In embodiments of any of these methods, harA disruption techniques, for example, those described hereinbelow or others known in the art, may be used to produce a control (sensitive) organism from a resistant organism, wherein resistance is mediated by harA. Similarly, the general applicability of the disruption techniques disclosed herein allows screening to be performed using any organism in which drug resistance is mediated by expression of a harA gene.

This invention also relates to a method for the identification of harA-mediated resistance in an organism or a panel of organisms which is resistant to a hygromycin A, a virginiamycin, a lankacidin or a streptogramin or streptogramin combination, e.g., Synercid, which comprises isolating an organism which is resistant to a drug, producing a plasmid suitable for disrupting a harA gene in the organism, transforming the organism with the plasmid, contacting the transformed organism with the drug, and measuring in the transformed organism an increase in susceptibility to the drug compared to a control organism, thereby identifying the organism as exhibiting harA-mediated drug resistance. As used herein, the phrase "increase in susceptibility" is equivalent to a "decrease in resistance" as that phrase is used herein. In an embodiment, the plasmid suitable for disrupting the harA gene comprises an internal fragment of the *B*. *subtilis* harA gene excised using EcoRV and EcoRI. In another embodiment, the plasmid suitable for disrupting the harA gene comprises an internal fragment of an *E. faecalis har*A gene generated by PCR using the sense and antisense primers of SEQ ID NO:5 and SEQ ID NO:6, respectively. In a preferred embodiment thereof, the plasmid comprises the *E. coli* vector pVA891, which carries an *erm* determinant that expresses in *E. coli* and *E. faecalis.* In an embodiment, the control is the resistant organism (i.e., not transformed with the disruption plasmid). In another embodiment, the control is the resistant organism transformed with a plasmid lacking the disruption construct.

This invention relates to the use of a hygromycin A-resistant strain of E. faecalis to determine whether an antibacterial agent is effective in treating organisms which exhibit harA-mediated drug resistance. In an embodiment, the hygromycin A-resistant strain of E. faecalis is the strain OG1X (ATCC Accession No. PTA-3272).

This invention also relates to the use of a hygromycin A-resistant strain of B. subtilis to determine whether an antibacterial agent is effective in treating organisms which exhibit harA-mediated drug resistance. In an embodiment, the hygromycin A-resistant strain of B. subtilis is the strain JH642 (ATCC Accession No. PTA-3271).

This invention also relates to a method of screening an antibacterial agent to determine whether the agent is effective against a first organism exhibiting harA-mediated drug-resistance, which comprises (a) measuring MIC₁ and MIC₂ and (b) calculating the ratio MIC₁/MIC₂, wherein a ratio of less than about four identifies the agent as effective against the first organism; wherein MIC₁ is the MIC of the agent against a second organism which exhibits harA-mediated drug resistance; and wherein MIC₂ is the MIC of the agent against a third organism not exhibiting harA-mediated drug resistance. In an embodiment of the method, the first organism is E. faecalis, E. faecium, Staphylococcus aureus, Streptococcus pyogenes, Streptococcus pneumoniae, Haemophilus influenzae, or Serpulina hyodysenteriae. Resistant organisms may be identified by e.g., surveillance of strains infecting animal populations, including humans, or screening of laboratory strains; harA-mediated resistance is identified as described herein, e.g., by the conferral of susceptibility by disruption of the harA gene in the resistant organism. In another embodiment, the drug is a hygromycin A, a virginiamycin, a lankacidin, a streptogramin or streptogramin combination, e.g., Synercid. In another embodiment of the method, the first organism and the second organism are the same organism. In another embodiment of the method, the second organism is the E. faecalis strain OG1X (ATCC Accession No. PTA-3272) or the B. subtilis strain JH642 (ATCC Accession No. PTA-3271). In another embodiment, the second organism is a revertant of an organism containing a disrupted harA gene. In an embodiment thereof, the second organism is a revertant of the E. faecalis strain OG1X harA::ERM (ATCC Accession No. PTA-2550) or the B. subtilis strain JH642 expZ::CAT (ATCC Accession No. PTA-2480). In another embodiment of the method, the third organism is a strain of the second organism in which harA expression is disrupted; in a preferred embodiment thereof, the first organism and the second organism are the same organism. In another embodiment, the third organism is the E. faecalis strain OG1X harA::ERM (ATCC Accession No. PTA-2550) or the B. subtilis strain JH642 expZ::CAT (ATCC Accession No. PTA-2480). In still another embodiment, the second organism is the E. faecalis strain OG1X (ATCC Accession No. PTA-3272) and the third organism is OG1X harA::ERM (ATCC Accession No. PTA-2550). In still another preferred embodiment, the second organism is the B. subtilis strain JH642 (ATCC Accession No. PTA-3271) and the third organism is the JH642 expZ::CAT (ATCC Accession No. PTA-2480). In other embodiments, for example, the second and third organisms may be selected resistant and susceptible strains of an organism, respectively, which are particularly suitable for screening antibacterial agents, while the first organism may be a related but resistant strain of the organism isolated from, e.g., an infected animal population. In other embodiments, for example, the third organism is a naturally-susceptible strain of the first organism or a knockout or disruption mutant of the first organism. In other embodiments, the ratio MIC₁/MIC₂ is less than about ten.

In embodiments of any of the above processes, the harA polypeptide is any of the above-identified harA polypeptides, including but not limited to a polypeptide comprising an amino acid sequence at least 75% identical to the amino acid sequence shown in SEQ ID NO:2 or SEQ ID NO:4, or at least 95% identical or 100% identical to the sequence as shown in SEQ ID NO:2 or SEQ ID NO:4, or any of the variants, fragments described herein.

This invention relates to an isolated nucleic acid encoding a harA polypeptide. In an embodiment, the nucleic acid is isolated from *E. faecalis,* and is preferably isolated from the *E. faecalis* strain OG1X (ATCC Accession No. PTA-3272). In another embodiment, the nucleic acid is isolated from *B. subtilis,* and is preferably isolated from the *B. subtilis* strain JH642 (ATCC Accession No. PTA-3271).

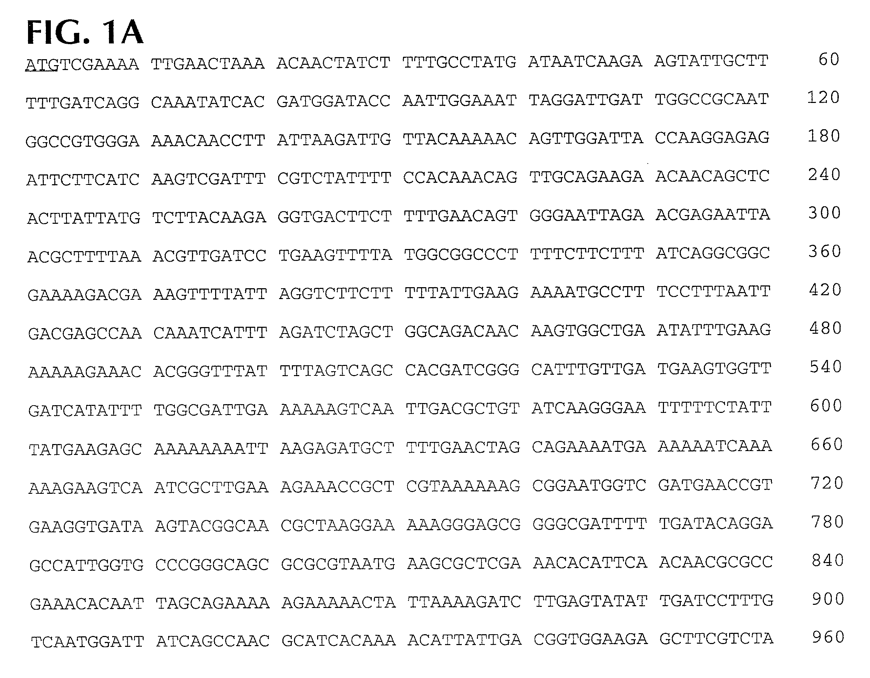

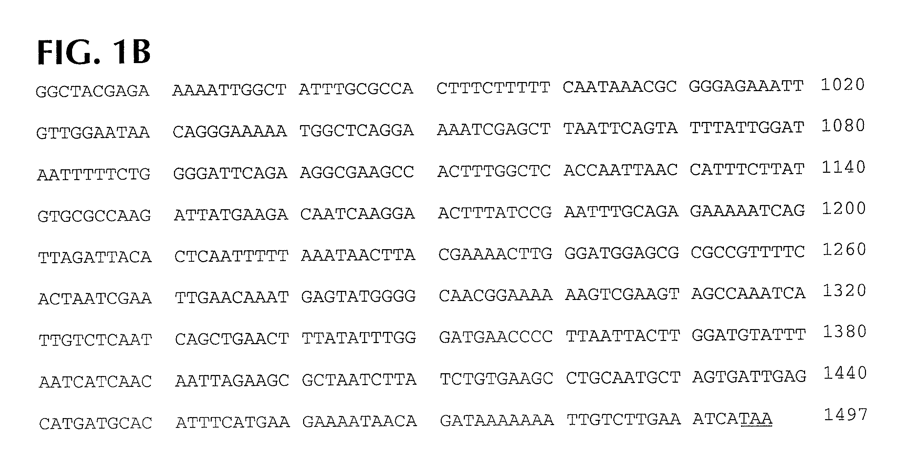

This invention relates to an isolated nucleic acid encoding a harA polypeptide, wherein the nucleic acid comprises a nucleotide sequence selected from the group consisting of: (a) a nucleotide sequence which is at least 75% identical to the nucleotide sequence shown in Figure 1 (SEQ ID NO:1); (b) a nucleotide sequence complementary to the nucleotide sequence of (a); and (c) a nucleotide sequence that is degenerate to the nucleotide sequence of (a) or (b). In an embodiment, the nucleic acid comprises a nucleotide sequence which is at least 85% identical to the nucleotide sequence shown in Figure 1 (SEQ ID NO:1). In a preferred embodiment, the nucleotide sequence consists essentially of the nucleotide sequence shown in Figure 1 (SEQ ID NO:1). In another embodiment, the nucleic acid comprises a nucleotide sequence encoding a harA polypeptide comprising an amino acid sequence consisting essentially of the amino acid sequence shown in Figure 2 (SEQ ID NO:2). In still another embodiment, the nucleotide sequence encodes the amino acid sequence shown in Figure 2 (SEQ ID NO:2). In yet another embodiment, the nucleotide sequence is at least 75% identical to, but less than 100% identical to, the nucleotide sequence shown in Figure 1 (SEQ ID NO:1). In still another embodiment, the nucleotide sequence is the nucleotide sequence shown in Figure 1 (SEQ ID NO:1).

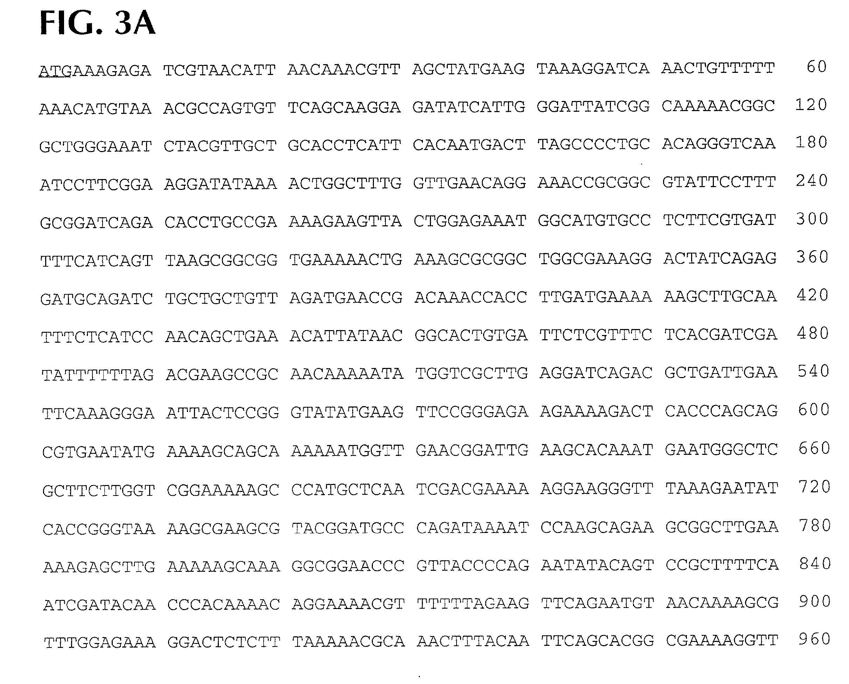

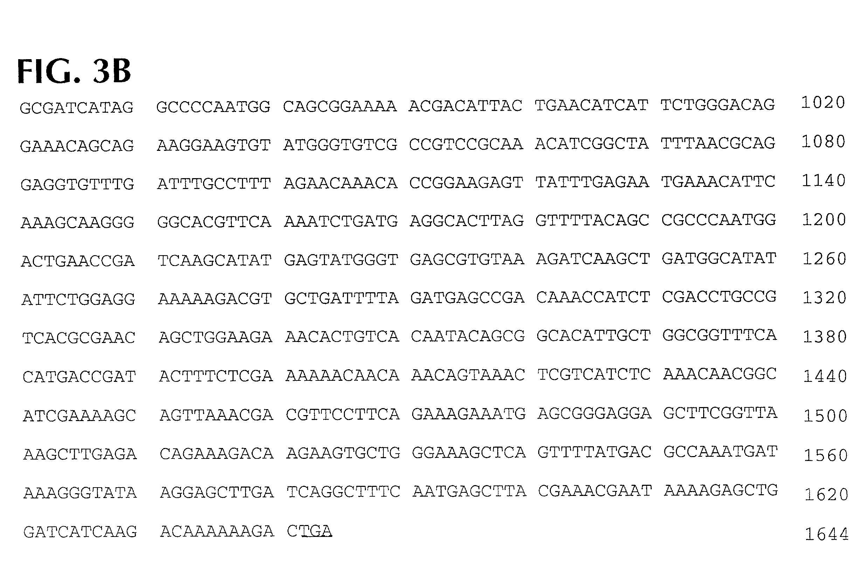

This invention relates to an isolated nucleic acid encoding a harA polypeptide, wherein the nucleic acid comprises a nucleotide sequence selected from the group consisting of: (a) a nucleotide sequence which is at least 75% identical to the nucleotide sequence shown in Figure 3 (SEQ ID NO:3); (b) a nucleotide sequence complementary to the nucleotide sequence of (a); and (c) a nucleotide sequence that is degenerate to the nucleotide sequence of (a) or (b). In an embodiment, the nucleic acid comprises a nucleotide sequence which is at least 85% identical to the nucleotide sequence shown in Figure 3 (SEQ ID NO:3). In a preferred embodiment, the nucleotide sequence consists essentially of the nucleotide sequence shown in Figure 3 (SEQ ID NO:3). In another embodiment, the nucleic acid comprises a nucleotide sequence encoding a harA polypeptide comprising an amino acid sequence consisting essentially of the amino acid sequence shown in Figure 4 (SEQ ID NO:4). In still another embodiment, the nucleotide sequence encodes the amino acid sequence shown in Figure 4 (SEQ ID NO:4). In yet another embodiment, the nucleotide sequence is at least 75% identical to, but less than 100% identical to, the nucleotide sequence shown in Figure 3 (SEQ ID NO:3). In still another embodiment, the nucleotide sequence is the nucleotide sequence shown in Figure 3 (SEQ ID NO:3).

In this application, the term "nucleic acid" includes polynucleotides generally, which may include genomic DNA and cDNA, synthetic DNA, and RNA, any of which may contain introns, or other nucleic acid to be removed prior to transcription or translation as required, or alternatively spliced or otherwise processed, and all of the nucleic acids referred to herein may comprise synthetic polynucleotides or polynucleotides containing one or more non-naturally occurring nucleotides, all of which are well known in the art.

In this application, a nucleic acid is "degenerate" with respect to a reference nucleic acid, when the nucleic acid differs from the reference nucleic acid by the inclusion of one or more nucleotide substitutions or alternative codons, but encodes a polypeptide having the same amino acid sequence as the polypeptide encoded by the reference nucleic acid. This invention further relates to all the sequences degenerate to those disclosed herein. It is expected that one of ordinary skill in the art may routinely determine those substitutions and alternate codons which are preferred in particular organisms, as codon usage tables are widely available to the public. It is to be further understood that the term "degenerate" encompasses alternative codon usage, including alternate translation initiation and termination codons, and generally alternative codon usage as is known to occur in different organisms, which is well-known in the art and described further herein.

The term "nucleic acid" as used herein encompasses polynucleotides that include a sequence encoding a polypeptide of the invention, particularly a bacterial polypeptide and more particularly a polypeptide of E. faecalis harA having the amino acid sequence set out in Figure 2 (SEQ ID NO: 2). The term also encompasses polynucleotides that include a single continuous region or discontinuous regions encoding the polypeptide (for example, interrupted by integrated phage or an insertion sequence or editing) together with additional regions, that also may contain coding and/or non-coding sequences.

The degree (%) of identity of two nucleotide sequences is as defined herein, wherein preferred parameters for polynucleotide comparison include the following: Algorithm: Needleman and Wunsch, J. Mol Biol. 48: 443-453 (1970) using Comparison matrix: matches=+10, mismatch=0 ; Gap Penalty: 50 ; and Gap Length Penalty: 3. This algorithm is available as the "gap" program from Genetics Computer Group, Madison, Wis. These are the default parameters for nucleic acid comparisons.

Preferred polynucleotide embodiments further include an isolated nucleic acid comprising a nucleotide sequence having at least 75, 80, 85, 90, 95, 97 or 100% identity to a reference sequence. In an embodiment, the reference sequence is SEQ ID NO: 1. In another embodiment, the reference sequence is SEQ ID NO:3. In an embodiment, the nucleic acid comprises a sequence identical to the sequence of SEQ ID NO: 1. In another embodiment, the nucleic acid comprises a sequence identical to the sequence of SEQ ID NO: 3. In yet another embodiment, the nucleic acid comprises a sequence including up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. In a preferred embodiment, the integer number is less than 50. In more preferred embodiments, the integer number is less than 40, less than 30 or less than 10. Alterations of a polynucleotide sequence encoding the polypeptide of SEQ ID NO: 2 may create nonsense, mis-sense or frameshift mutations in this coding sequence and thereby alter the polypeptide encoded by the polynucleotide following such alterations.

A "variant(s)" nucleic acid as the term is used herein, is a nucleic acid that differs from a reference nucleic acid but retains essential properties. A typical variant of a nucleic acid differs in nucleotide sequence from another, reference nucleic acid. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence. Generally, differences are limited so that the sequences of the reference polypeptide and the variant are closely similar overall and, in many regions, identical. A variant of a nucleic acid may be naturally occurring such as an allelic variant, or it may be a variant that is not known to occur naturally. Non-naturally occurring variants of nucleic acids may be made by mutagenesis techniques, by direct synthesis, and by other recombinant methods known to skilled artisans. A variant may include a marker sequence, such as a sequence encoding a hexa-histidine peptide, as provided in the pQE vector (Qiagen, Inc.) and described in Gentz et al., Proc. Natl. Acad. Sci., (USA) 86: 821-824 (1989), or an HA tag (Wilson et al., Cell 37: 767 (1984).

The invention also relates to harA nucleic acids encoding derivatives, analogs and variants of harA-encoded proteins, and harA antisense nucleic acids. As is readily apparent, as used herein, a "nucleic acid encoding a fragment or portion of a harA-encoded protein" shall be construed as referring to a nucleic acid encoding only the recited fragment or portion of the harA-encoded protein and not the other contiguous portions of the harA-encoded protein as a continuous sequence.

This invention relates to any of the harA nucleic acids as described herein, which is in operative association with a heterologous promoter. In an embodiment thereof, the nucleic acid further comprises an origin of replication active in a prokaryotic cell. In a preferred embodiment thereof, the nucleic acid further comprises a recombinant vector comprising any of the harA nucleic acids described herein. In a more preferred embodiment, the vector is selected from the vectors pMP, pGEM-T, pVA891, pUC8, pUC9, pBR322, pBR329, pPL, pKK223 and pQE50. This invention also relates to a cell comprising any of the above vectors.

This invention relates to a vector which is pMP-bacA1-1 (ATCC Accession No. PTA-2551, deposited September 26, 2000).

This invention also relates to a vector which is pGEM-T/harA (ATCC Accession No. PTA-2552, deposited September 26, 2000).

This invention relates to any of the nucleic acids described herein which further comprises an origin of replication active in a eukaryotic cell. In an embodiment thereof, the nucleic acid further comprises a recombinant vector. In preferred embodiments thereof, the vector is pcDNA-3, pcEXV, a yeast vector such as Yep13, Yip5, pYAC such as pYAC3, and fusion protein vectors such as pMal, pTrxFus, and pGEX, all of which are well known in the art. In a preferred embodiment thereof, the invention relates to a eukaryotic cell comprising the vector. In a more preferred embodiment, the eukaryotic cell is a CHO, COS, COS-7, NIH-3T3, HEK-293, MDCK or LM(tk-) cell, or an insect cell such as an Sf-9 or Sf-21 cell, or a Xenopus cell such as an oocyte or a Xenopus cell line, all of which are well-known in the art.

This invention relates to an isolated nucleic acid encoding a harA polypeptide and further comprising a nucleotide sequence comprising a chloramphenicol resistance insertion. In an embodiment thereof, the nucleic acid further comprises a recombinant vector. In a preferred embodiment thereof, the invention relates to a cell comprising the recombinant vector. The invention relates still further to a cell which is the Bacillus subtilis strain JH642 expZ::CAT (ATCC Accession No. PTA-2480, deposited September 26, 2000), which comprises a chloramphenicol resistance insertion.

This invention relates to an isolated nucleic acid encoding a harA polypeptide and further comprising a nucleotide sequence comprising an erythromycin resistance insertion. In an embodiment thereof, the nucleic acid further comprise a recombinant vector. In a preferred embodiment, the invention relates to a cell comprising the recombinant vector. The invention relates still further to a cell which is the Enterococcus faecalis strain OG1X harA::ERM (ATCC Accession No. PTA-2550, deposited September 26, 2000), which comprises an erythromycin resistance insertion.

The deposits in the American Type Culture Collection ("ATCC") described herein have been made under the terms of the Budapest Treaty on the International Recognition of the Deposit of Micro-organisms for Purposes of Patent Procedure. The deposits described herein are provided merely as convenience to those of skill in the art, as the claimed nucleic acids, vectors, plasmids and cells are readily obtainable, and related processes, methods and uses thereof are all readily performed, using the nucleotide and amino acid sequences disclosed herein and methods well-known in the art.

The Enterococcus faecalis strain OG1X is publicly available from the University of Oklahoma (contact Enterococcus@ouhsc.edu), Biomedical Research Center Building, Rm. 356, The University of Oklahoma, 975 NE 10th Street, Oklahoma City, OK 73104, USA. Strain OG1X was deposited with the ATCC on April 12, 2001 and has been accorded ATCC Accession No. PTA-3272. The Bacillus subtilis strain JH642 is publicly available from the Bacillus Genetic Stock Center (BGSC) at the Ohio State University, contact zeigler.1@osu.edu or Dr. D.R. Zeigler, Department of Biochemistry, The Ohio State University, 484 West Twelfth Avenue, Columbus, OH 43210, USA. Strain JH642 was also deposited with the ATCC on April 12, 2001 and has been accorded ATCC Accession No. PTA-3271.

A nucleic acid of the invention may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide. A nucleic acid of the invention may also encode a mature protein plus a fusion protein partner which is useful for identification, isolation or detection of the harA polypeptide. Fusion protein partners are well-known in the art.

The invention relates to a harA nucleic acid as described herein which further comprises a nucleotide sequence encoding a fusion partner such that expression of the nucleic acid results in a fusion polypeptide comprising the fusion partner and a harA polypeptide.

This invention relates to the use of a nucleic acid encoding a harA polypeptide to identify an organism containing a harA gene. Such uses provide methods for surveillance of individual animals and populations of animals, especially mammals, and more especially humans, to identify, isolate and treat individuals or populations suffering from or vulnerable to infection by an organism containing or expressing a harA gene, particularly Enterococcus, Streptococcus or Haemophilus organisms as described herein.

This invention relates to a method for identifying a drug-resistant infectious organism, which comprises isolating nucleic acid from the organism, and determining whether a harA nucleic acid hybridizes to the nucleic acid from the organism, under moderately or highly stringent conditions. In an embodiment, the infectious organism is isolated from an infected animal. In another embodiment, the harA nucleic acid comprises at least 15 nucleotides, preferably from about 15 to about 50 nucleotides. In another embodiment, the harA nucleic acid used in a hybridization assay is from about 50 to about 150 nucleotides. In a preferred embodiment thereof, the nucleic acid is from about 90 to about 120 nucleotides. The invention encompasses probes used both for hybridization assays, e.g., FISH (fluorescence in situ hybridization) and for amplification assays, e.g., PCR, as needed to identify harA nucleic acids and related nucleic acids. These are just two examples of the many methods of using nucleic acid probes which are well-known in the art.

This invention provides an isolated nucleic acid which hybridizes under moderately stringent conditions to a nucleic acid comprising the nucleotide sequence shown in Figure 1 (SEQ ID NO:1). In an embodiment, the nucleic acid comprises from about 15 to about 40 nucleotides.

This invention provides an isolated nucleic acid which hybridizes under moderately stringent conditions to a nucleic acid comprising the nucleotide sequence shown in Figure 3 (SEQ ID NO:3). In an embodiment, the nucleic acid comprises from about 15 to about 50 nucleotides. In a preferred embodiment, the nucleic acid comprises at least about 30 nucleotides. In another preferred embodiment, the nucleic acid comprises less than about 50 nucleotides. In an embodiment, the above nucleic acids comprise at least 30 nucleotides.

"Stringent conditions" and "stringent hybridization conditions" mean hybridization will occur only if there is at least 95% and preferably at least 97% identity between the sequences. Examples of hybridization conditions useful in the practice of the invention are described further herein.

Nucleic acids of the invention that are oligonucleotides derived from the sequences of SEQ ID NOS: 1 and/or 3 may be used in the methods and processes herein as described, but preferably for PCR, to determine whether or not the nucleic acids identified herein in whole or in part are transcribed in bacteria in infected tissue. It is recognized that such sequences will also have utility in diagnosis of the type of infection and the stage of infection the pathogen has attained. It is further recognized that the detection of harA nucleic acid sequences in bacteria and/or infected tissue has utility in the diagnosis of drug-resistance.

Nucleic acids of the invention as discussed above, may be used as a hybridization probe for RNA, cDNA and genomic DNA to isolate full-length cDNAs and genomic clones encoding harA polypeptides and to isolate cDNA and genomic clones of other genes that have a high sequence similarity to a harA gene. Such probes generally will comprise at least 15 bases. Preferably, such probes will have at least 30 bases and may have at least 50 bases. Particularly preferred probes will have at least 30 bases and will have 50 bases or less.

The invention also provides a nucleic acid consisting essentially of a polynucleotide sequence obtainable by screening an appropriate genomic, cDNA or other library containing the complete gene for a nucleotide sequence set forth in SEQ ID NO: 1 (or SEQ ID NO:3) under stringent hybridization conditions with a probe having the sequence of said polynucleotide sequence set forth in SEQ ID NO: 1 (or SEQ ID NO:3) or a fragment thereof; and isolating said DNA sequence. Fragments useful for obtaining such a polynucleotide include, for example, probes and primers described elsewhere herein.

For example, the coding region of a harA gene may be isolated by screening using the DNA sequence provided in SEQ ID NO: 1 to synthesize an oligonucleotide probe. A labeled oligonucleotide having a sequence complementary to that of a gene of the invention is then used to screen a library of cDNA, genomic DNA or mRNA to determine which members of the library the probe hybridizes to. Libraries containing nucleic acids from pathogenic organisms including cDNA and genomic DNA libraries useful for homology cloning or expression cloning, are readily prepared by methods well known in the art.

The invention also relates to a kit for detection of *E. faecalis,* harA-resistant *E. faecalis,* an *E. faecalis* specific amino acid or nucleotide sequence, or an anti-*E. faecalis* antibody, comprising a container that has therein at least a polypeptide, nucleic acid, or antibody of the invention. In an embodiment, the container contains at least a nucleic acid which hybridizes under highly stringent conditions to a nucleic acid comprising the nucleotide sequence shown in Figure 1 (SEQ ID NO:1). In a preferred embodiment thereof, the nucleic acid comprises 15-40 nucleotides. In another embodiment, the container contains at least a nucleic acid which hybridizes under highly stringent conditions to a nucleic acid comprising the nucleotide sequence shown in Figure 3 (SEQ ID NO:3). In a preferred embodiment thereof, the nucleic acid comprises 15-40 nucleotides.

The term "open reading frame" or "ORF" means the coding region of a nucleic acid, i.e., comprising a start and stop codon and a plurality of intervening nucleotide triplets or "codons" encoding naturally occurring amino acids.

"Host cell" is a cell which has been transformed or transfected, or is capable of transformation or transfection by an exogenous polynucleotide sequence.

The following examples are illustrative only, and are not intended to limit the scope of the present invention.

### BRIEF DESCRIPTION OF DRAWINGS

Figure 1 shows the nucleotide sequence of E. faecalis encompassing an open reading frame encoding harA (SEQ ID NO: 1). The start (ATG) and stop (TAA) codons are underlined, and the putative ABC domains are also underlined.
Figure 2 shows the amino acid sequence of the harA polypeptide encoded by the harA nucleic acid shown in Figure 1 (SEQ ID NO: 2). Sequences with a single underline (e.g., GlyArg) or a double underline (e.g., AspGlu) represent the "Walker A box" and "Walker B box" ABC protein motifs, respectively. See, Walker J.E., et al. EMBO J. 1:945-951 (1982). Sequences with a dotted underline (e.g., LeuSer) represent an ABC "signature motif" and sequences with a dashed underline (e.g., ValSer) represent an ABC "downstream motif". See, Quentin et al. J. Mol. Biol. 287:467-484 (1999).
Figure 3 shows the nucleotide sequence of B. subtilis encompassing an open reading frame encoding expZ (harA) (SEQ ID NO: 3). The start (ATG) and stop (TGA) codons are underlined.
Figure 4 shows the amino acid sequence of the harA polypeptide encoded by the B. subtilis expZ (harA) nucleic acid shown in Figure 3 (SEQ ID NO: 4). The Walker A, Walker B, signature and downstream motifs are indicated in the same way as in Figure 2.

### DETAILED DESCRIPTION OF THE INVENTION

Applicants have discovered strains of *E. faecalis* and *B. subtilis* which are resistant to the antibacterial agent hygromycin A and other, structurally unrelated agents, and have identified a previously uncharacterized endogenous gene product designated "harA", which confers that resistance. The isolation and characterization of harA nucleic acids and polypeptides are described below. All patents, patent applications, and publications cited herein are hereby incorporated by reference in their entireties.

### Isolation of harA nucleic acids

Applicants observed that *Bacillus subtilis* strain JH642 was naturally resistant to a nonpolar hygromycin A analogue. Hygromycin A analogues are well-known in the art; however, examples of hygromycins suitable for resistance screening may be found in e.g., PCT International Applications WO00/32616, WO99/57125 and WO99/57127. In an effort to identify putative resistance determinants, applicants used a heterologous functional cloning strategy with a *Bacillus subtilis* genomic plasmid library to identify genes which, when amplified on a multicopy plasmid, would give rise to a hygromycin-resistant phenotype in a sensitive *Staphylococcus aureus* strain. This selection, described in detail below, led to the identification of a previously uncharacterized gene, *expZ,* whose product was predicted to encode an ATP binding cassette (ABC) protein with similarity to the orthologous staphylococcal proteins, *vga* and *msr*A, which prevent the intracellular accumulation of virginiamycin and erythromycin, respectively. Overexpression of expZ is sufficient to confer a high-level of resistance to a number of structurally unrelated agents in *S*. *aureus.* Detailed procedures, which are not intended to be limiting as to the invention described herein, are provided below.

### Construction of a plasmid library and cloning of the Bacillus subtilis expZ gene

To identify genes whose products might be important in establishing the intrinsic level of resistance to hygromycins, applicants screened a recombinant library of *B. subtilis* chromosomal DNA segments cloned into the *E. coli-S. aureus* shuttle vector pMP20 for *S. aureus* transformants with increased levels of resistance to a hygromycin analogue, as follows:

Chromosomal DNA was extracted from a 100 mL culture of *B. subtilis* strain JH642 grown in L broth, partially digested with *Sau*3A and fractionated by electrophoresis in a 1% agarose gel. A fraction corresponding in size to 2-4 kb was purified and used for ligation with pMP20 that had been digested with *Bam*HI and dephosphorylated with alkaline phosphatase. Ligated DNA was used to transform *E. coli* DH5α by electroporation, with selection for ampicillin resistance. Transformant colonies were pooled; the plasmid DNA mixture was extracted and used to transform *S. aureus* RN4220 by electroporation with selection on plates containing erythromycin (2 µg/mL) and 2 µg/mL of a nonpolar hygromycin A analogue, 3-(4-((2S,4S,5R)-5-(3-(2,4-dichloro-phenoxy)-1-methyl)-(1E)-propenyl)-4-hydroxy-tetrahydro-furan-2-yloxy)-3-hydroxy-phenyl)-2-methyl-N-((3aS,4R,5R,6S,7R,7aR)-4,6,7-trihydroxy-hexahydro-benzo(1,3)dioxol-5-yl)-(2E)-acrylamide, which is Example 68 of WO 00/32616 and referred to herein as "Compound 1".

Vector-transformed *S*. *aureus* cells were sensitive to this level of Compound 1. Plasmid segregation and backcross experiments confirmed that plasmids harboring DNA segments from the *B. subtilis* chromosome conferred hygromycin A resistance in *S. aureus.* The MIC for Compound 1 was increased in these strains from ≤0.2 µg/mL for pMP20 transformants to 50 µg/mL for cells transformed with the recombinant plasmids. Similar increases in resistance were also observed for lankacidin, virginiamycin and Synercid®.

DNA sequence analysis of the ends of the several cloned inserts and subsequent searching of the *B. subtilis* genome sequence database (http://genolist.pasteur.fr/SubtiList/) revealed that the resistance phenotype was associated with the presence of the "expZ" gene on each plasmid. The *B. subtilis* gene expZ is a previously uncharacterized ORF with similarity to members of the ATP binding cassette (ABC) protein family including the S. *aureus* Vga and *S. epidermidis* MsrA proteins. ExpZ, *vga* and *msrA* all lack transmembrane domains or associated transmembrane partners and each consists of two ABC subunits arranged in tandem, forming an ABC-ABC covalent dimer. The similarity is highest in the regions that include the putative nucleotide binding domains. However, homology with some yeast ABC proteins suggests that *exp*Z could code for a novel ribosomal protein involved in translation of mRNA. Because of the low level of sequence similarity between expZ and other proteins, and the low similarity among various proteins containing ABC's, the intrinsic activity of the expZ protein can not be determined based on its sequence. However, applicants have demonstrated that expression of the harA gene confers resistance to hygromycin A and other compounds, e.g., virginiamycin, lankacidin and Synercid®, and have shown that harA-specific disruption of the gene re-establishes susceptibility to hygromycin A (see below). Accordingly, applicants propose renaming the gene "harA" for "Hygromycin A Resistance".

### Mutant harA nucleic acids

### Generation of a B . subtilis harA::CAT insertion mutant

A *B. subtilis* strain expressing a harA::CAT insertion mutant was produced by single-crossover recombination using integrative plasmid pJPM1. Plasmid pJPM1 is a pSK⁻ derivative that carries the *cat* gene of *S*. *aureus* plasmid pC194 that confers chloramphenicol resistance in *B. subtilis.* Plasmid pJPM1 does not contain an origin of replication for *B. subtilis* and cannot replicate in the host. However, if any DNA fragment providing a region of homology with the *B. subtilis* chromosome is inserted into this plasmid, it acquires the ability to transform *B. subtilis* competent cells to *cat* resistance at high frequency. The integration occurs via a Campbell-type (single crossover) recombination event that results in the duplication of the cloned region at either end of the vector. Reversal of the integration event leads to precise excision of the plasmid and restoration of gene structure on the chromosome. An internal fragment of the *B. subtilis* harA gene was excised using EcoRV and EcoRI and subcloned into compatible sites in pJPM1 creating plasmid pJPM1-expZ1. Plasmid pJPM1-expZ1 was used to transform the naturally competent *B. subtilis* strain JH642 (hygromycin-resistant) and transformants selected using chloramphenicol (5 µg/mL). A *har*A::*CAT* insertion mutant was confirmed by PCR and the strain was designated "JH642 expZ::CAT." Strain JH642 expZ::CAT was hypersensitive to hygromycin analogues and other unrelated antibiotics including lankacidin and virginiamycin. Strain JH642 expZ::CAT was deposited with the ATCC on September 26, 2000, and accorded Accession No. PTA-2480.

### Generation of a Enterococcus faecalis harA::ERM insertion mutant

A PCR product corresponding to an internal segment of the *E. faecalis* harA gene was generated to obtain pVA891-expZ by using the *E. coli* vector pVA891, which carries an *erm* determinant that expresses in *E. coli* and *E. faecalis.* Briefly, internal *har*A primers were used (sense: 5'- TCTAGATATCACGATGGATACC-3' - SEQ ID NO: 5); (antisense: 5'-TCTAGATTGCCGTACTTATCACC-3' - SEQ ID NO: 6) with *E*. *faecalis* genomic template DNA to generate a 665 bp PCR product. PCR-generated fragments were purified using the QlAquick PCR purification kit and cloned into the T-A cloning plasmid pGem-T Easy. The fragment was subsequently excised using *Eco*RI, gel purified using the S&S Elu-Quik kit, and cloned into the *Eco*RI site of pVA891. The ligation was transformed in *E. coli* DH5α with selection for erythromycin resistance (200 µg/mL); resulting in pVA891-expZ. The plasmid pVA891-expZ was introduced by electroporation into *E. faecalis* OG1X (hygromycin resistant), where integrants occurring via homologous recombination were selected using erythromycin (20 µg/mL). The *har*A::*erm* knockout strain was confirmed by PCR and designated "OG1X harA::erm." Strain OG1X harA::erm was hypersensitive to nonpolar hygromycin A analogues, lankacidin, virginiamycin and Synercid®. Revertants (i.e., resistant strains) could be obtained by growing for several generations in the absence of erythromycin and identified by screening for resistance to hygromycin A and sensitivity to erythromycin. Strain OG1X harA::erm was deposited with the ATCC on September 26, 2000, and was accorded Accession No. PTA-2550.

### Purification of recombinantly-produced proteins in E. coli

For effective recombinant protein production, a gene must be linked to a promoter sequence. Promoters for use in bacterial systems also will contain a Shine-Dalgarno sequence, operably linked to the DNA encoding the desired polypeptides. In a preferred process for protein purification a gene is modified at the 5' end, or at some other position, such that the encoded protein incorporates several histidine residues (viz. "histidine tag"). This histidine tag allows the use of immobilized metal ion affinity chromatography (IMAC), a single step protein purification method described in US Pat. No. 4,569,794. The IMAC method allows isolation of substantially pure protein starting from a crude cellular extract.

### Production of vectors for expressing E. faecalis and B. subtilis harA protein in E. coli

An expression vector suitable for expressing harA proteins in *E. coli* was prepared. The *B. subtilis* harA coding sequence was PCR cloned using forward primer B-EXPZ-EF2 (5'-CATATGAAAGAGATCGTAACATTAACAAACG-3'; SEQ ID NO:7) and reverse primer B-EXPZ-ER2 (5'-GGATCCTTAGTCTTTTTTGTCTTGATGATCC-3'; SEQ ID NO:8) using *B. subtilis* strain JH642 genomic DNA as template. The *E. faecalis har*A coding region was amplified using primers E-EXPZ-EF1 (5'-CATATGTCGAAAATTGAAC-3'; SEQ ID NO:9) and E-EXPZ-ER1 (5'-GGATCCTTATGATTTCAAGAC-3'; SEQ ID NO:10) and *E. faecalis* strain OG1X genomic DNA as the template. The resulting PCR products were cloned into pGEM-T EASY® (Promega, Madison, WI) as described by the manufacturer. The resulting plasmid inserts were confirmed by DNA sequence analysis and subsequently evaluated for protein production as described below. This plasmid, designated "pGEM-T/harA", was deposited with the ATCC on September 26, 2000, and accorded ATCC Accession No. PTA-2552.

Coding regions containing the correct DNA sequence were excised from pGEM-T EASY® using enzymes *Nde*l and *Bam*HI, and cloned into the corresponding sites of pET16b (Novagen, Madison, WI). Plasmid pET16b contains an origin of replication (Ori), an ampicillin resistance gene (Amp) useful for selecting cells which have incorporated the vector following a transformation procedure. Expression plasmids were identified and transformed into the *E. coli* expression strain BL-21(DE3) (Novagen). The pET16b-harA expression constructs contain a bacteriophage T7 promoter and T7 terminator sequences in operable linkage to the harA coding region. The harA protein expressed from each construct is modified at the amino terminus to contain 6-10 histidine residues to simplify purification of the encoded protein product. Placement of histidine residues at the amino terminus of the encoded protein allows IMAC one-step protein purification.

### Recombinant expression and purification of a protein encoded by E. faecalis harA and B. subtilis harA genes

An expression vector that carries the harA from *B. subtilis* or *E. faecalis* genomes as disclosed herein, in which harA is operably-linked to an expression promoter and the bacteriophage T7 g10 ribosome binding site, was transformed into *E. coli* BL21(DE3) (hsdS gal λclts857 indλSam7*nin*5 *lac*UV5-T7 gene 1) using standard methods. Transformants, selected for resistance to ampicillin, were chosen by random and tested for the presence of vector by agarose gel electrophoresis using quick plasmid preparations or plasmid-specific PCR analysis. Colonies that contained the expression plasmid were grown in L broth and the protein product encoded by the plasmid-borne ORF was purified by IMAC, essentially as described in U.S. Pat. No. 4,569,794.

The *har*A proteins were expressed and purified as follows. Freshly transformed BL-21(DE3) cells containing a harA expression plasmid were grown on L plates containing 100 ug/mL ampicillin overnight at room temperature. The next morning, 5 mL of L broth was added to each plate, the plates scraped, and the resulting cell suspension used to inoculate 100 mL of L broth containing 100 ug/mL ampicillin to an OD600 of approximately 0.1. Cultures were grown at 37°C with vigorous shaking to an OD600 of -0.6. At this point, cultures were shifted to 30°C and heterologous protein production induced with 1.0 mM IPTG for an additional 3 hours. Cells were harvested by centrifugation at 5000 x g for 15 minutes and frozen at -70°C until purification. Cells were rapidly thawed at 37°C and resuspended in 10 mL of lysis buffer (25 mM Tris-Cl (pH 8.0), 250 mM NaCI, 10% glycerol, 5 mM imidazole). Lysozyme (Sigma, 100 ug/mL) and complete EDTA-FREE Protease Inhibitor (Boehringer, to 1X) were added, and the suspension incubated on ice for 1 hour. The cells were lysed by disruption in a French pressure cell at 16,000 psi. The lysates were collected by centrifugation and the supernatant was clarified by passage through a 0.45 uM filter. Two milliliters of 50% Ni²⁺-agarose slurry (Qiagen; Madison, WI), equilibrated in lysis buffer, was added to the extract, and incubated at 4°C overnight with gentle rocking. The next morning, the extract/bead mixture was packed in a 10 mL (0.8 x 4.0 cm) Bio-Rad disposable column. Unbound proteins and other materials were removed by washing the column with the wash buffer (25 mM Tris-CI (pH 8.0), 250 mM NaCI, 10% glycerol, 40 mM imidazole). The harA protein was eluted with 3 column volumes of elution buffer (25 mM Tris-CI (pH 8.0), 250 mM NaCI, 10% glycerol, 250 mM imidazole), dialyzed extensively against storage buffer (25 mM Tris-HCI (pH 8.0), 250 mM NaCl, 10% glycerol) and stored at -70°C.

### Antibiotic susceptibility testing

Quantitative assessment of resistance was performed by a broth microdilution method using cation-supplemented Mueller-Hinton broth containing antibiotics, the concentration of which varied in two-fold increments. The lowest concentration of antibiotic that completely inhibited growth was designated the MIC, or minimum inhibitory concentration.

Preferably, the microdilution assay is performed using cation-adjusted Mueller-Hinton broth according to NCCLS (National Committee for Clinical Laboratory Standards) guideline M31-A, Vol. 19, No. 11, "Performance standards for antimicrobial disk and dilution susceptibility tests for bacteria isolated from animals," June 1999 (ISBN 1-56238-377-9), which is herein incorporated by reference.

To confirm the role of *expZ* in conferring resistance to hygromycin A analogues and other compounds, applicants examined the effect of *B. subtilis* and *E. faecalis exp*Z expression and disruption on antibiotic resistance patterns. As shown in Table 1, the *exp*Z disruptions conferred a hygromycin A hypersensitivity phenotype compared with that of the *exp*Z⁺ or *harA*^{*+*} (i.e., unmodified, resistant) B. subtilis and E. faecalis strains. Also shown in Table 1 is the conferral of the "harA" phenotype on the normally drug-sensitive S. aureus RN4220 strain by transformation with a pMP plasmid containing B. subtilis harA.

It is important to note that the expected change in MIC from "sensitive" (susceptible) to "resistant" varies considerably among the tested antibacterial agents. For example, the increase in the MIC of the tested agents against a "sensitive" S. aureus strain compared to the same strain transformed with and expressing harA varies from about 8-fold for hygromycin A, to about 15-fold for Synercid, up to about a 500-fold difference for Compound 1. All of these changes are to be considered significant in methods of screening using a particular strain of an organism to an particular antibacterial agent. Similarly, testing of agents against naturally-resistant B. subtilis and E. faecalis strains shows that when the harA gene is disrupted, decreases in the MIC were variable, from greater than 4-fold (Compound 2 vs. E. faecalis OG1X) to about 16-fold (Hygromycin A vs. B. subtilis JH642), to greater than 100-fold (Lankacidin vs. E. faecalis OG1X, Compounds 1 and 2 vs. B. subtilis JH642). Again, all these differences are to be considered significant in methods of screening using a particular strain of an organism to an particular antibacterial agent. In particular, at least about a 4-fold difference should be observed in a microdilution method, due to the typical experimental uncertainty of this procedure. Compound 2, 5-deoxy-5[4-[(2,6-dideoxy-β-D-*erythro*-hexofuranos-5-ulos-1-yl)oxy]-3-hydroxyphenyl]-2-methyl-1-oxo-2-(E)-propenyl]amino]-1,2-O-methylene-D-neo-inositol, (E)-*O*-[(3-chlorophenyl)methyl]oxime, is a hygromycin A analogue, described in the PCT International application WO99/57127 (page 17, lines 28 - 30). Compound 3, 5-deoxy-5-[[3-[4-[(6-deoxy-β-D-*arabino*-hexofuranos-5-ulos-1-yl)oxy]-3-hydroxyphenyl]-2-methyl-1 -oxo-2-(E)-propenyl]amino]-1,2-*O*-methylene-D-neo-inositol, (E)-*O*-[(3-chlorophenyl)methyl]oxime, is a hygromycin A analogue described in the PCT International application WO 99/57125, page 65, Example 92.

Accordingly, by appropriate selection of a panel of antibacterial agents, such as is shown in Table 1, a variety of organisms can be screened to diagnose drug-resistance, and to determine whether the mechanism is harA-mediated. It is to be understood that the panel of antibacterial agents and organisms is expected to be more extensive in clinical practice, and that the extension of the methods shown herein is within the ordinary expertise of the art-skilled. These results confirmed that the *exp*Z gene is responsible for the intrinsic resistance of *B. subtilis* and *E. faecalis* to hygromycin A analogues and other structurally unrelated antibiotics including lankacidin, virginiamycin and Synercid®. Thus, the *exp*Z gene, described herein as "harA" represents a useful target as an intrinsic genetic system to assess the activity of hygromycin A-related antibiotics, and other agents as shown herein, against bacterial pathogens.

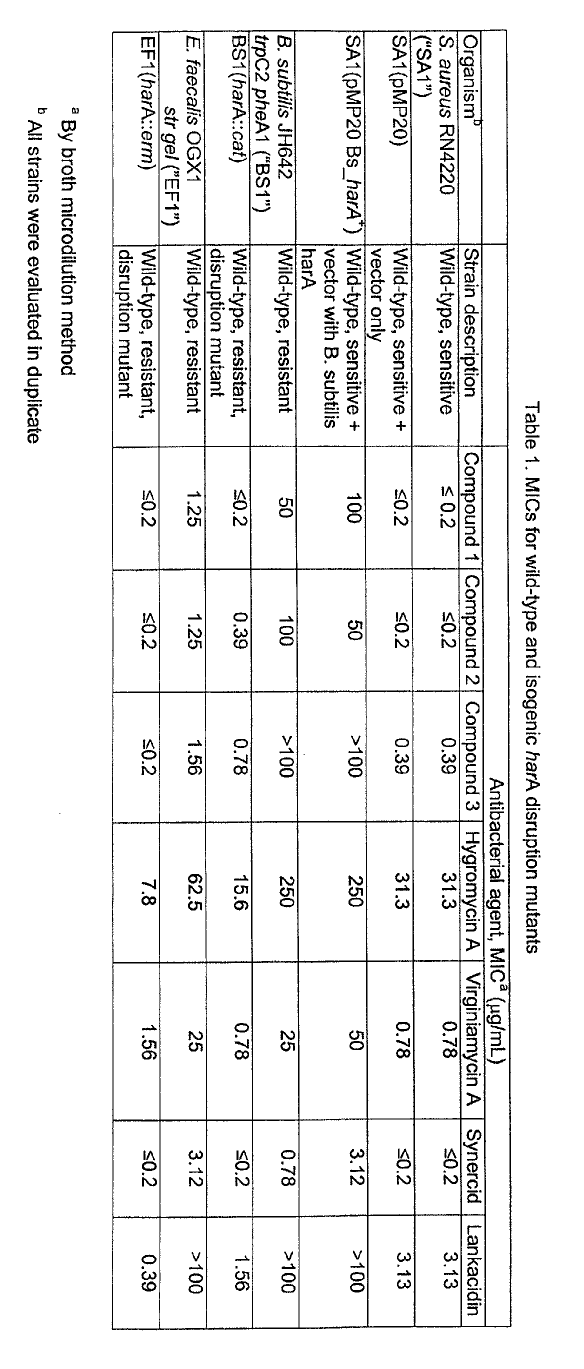

An isolated polynucleotide molecule of the present invention can have a nucleotide sequence derived from any strain of *E. faecalis,* but is preferably from the strain OG1X. Pathogenic strains of *E. faecalis* for use in practicing the present invention can be isolated from organs, tissues or body fluids of infected animals using isolation techniques as described below.

Production and manipulation of the polynucleotide molecules and oligonucleotide molecules disclosed herein are within the skill in the art and can be carried out according to recombinant techniques described, among other places, in Maniatis *et al.,* 1989, Molecular Cloning, A Laboratory Manual, Cold Spring Harbor Laboratory Press, Cold Spring Harbor, NY; Ausubel et al., 1989, Current Protocols In Molecular Biology, Greene Publishing Associates & Wiley Interscience, NY; Sambrook *et al.,* 1989, Molecular Cloning: A Laboratory Manual, 2d ed., Cold Spring Harbor Laboratory Press, Cold Spring Harbor, NY; Innis *et al.* (eds), 1995, PCR Strategies, Academic Press, Inc., San Diego; and Erlich (ed), 1992, PCR Technology, Oxford University Press, New York and all revisions of these references. Another useful description of PCR technology useful in practicing the invention is described in J. Sutcliffe et al., "Detection Of Erythromycin-Resistant Determinants By PCR", Antimicrobial Agents and Chemotherapy, 40(11), 2562-2566 (1996). The assay is preferably performed in microtiter trays.

References herein to the nucleotide sequences shown in SEQ ID NO: 1 and to substantial portions thereof, are intended to also refer to the corresponding nucleotide sequences and substantial portions thereof, respectively, as present in, e.g., the plasmid "pGEM-T/harA" which contains the E. faecalis harA gene and 5' upstream promoter region from E. faecalis strain OG1X. Plasmid pGEM-T/harA was deposited with the ATCC on September 26, 2000 and was accorded Accession No. PTA-2552.

In addition, references herein to the nucleotide sequences shown in SEQ ID NO: 1 and SEQ ID NO: 3, and to substantial portions thereof, are intended to also refer to the corresponding nucleotide sequences and substantial portions thereof, respectively, as present in, e.g., the plasmid "pMP-bac A1-1" containing the B. subtilis harA gene and 5' upstream promoter region from B. subtilis strain JH642. The plasmid pMP-bac A1-1 was deposited on September 26, 2000 with the ATCC and accorded Accession No. PTA-2551.

### Hybridization assays

Nucleic acid probe hybridization assays are well-known in the art; the conditions described herein are for illustrative purposes only. Hybridization under "moderately stringent conditions," as referred to herein, means, e.g., hybridization to filter-bound DNA in 0.5 M NaHPO₄, 7% sodium dodecyl sulfate (SDS), 1 mM EDTA at 65°C, and washing in 0.2xSSC/0.1% SDS at 42°C (see Ausubel *et al.* (supra), 1989, Current Protocols in Molecular Biology, Vol. I, Green Publishing Associates, Inc., and John Wiley & Sons, Inc., New York, at p. 2.10.3). Hybridization under "highly stringent conditions" as referred to herein means, e.g., hybridization to filter-bound DNA in 0.5 M NaHPO₄, 7% SDS, 1 mM EDTA at about 60°C for nucleic acids comprising about 23 nucleotides, about 55°C for nucleic acids comprising about 20 nucleotides, and about 48°C for nucleic acids comprising about 17 nucleotides, with washing in 0.1xSSC/0.1% SDS at 68°C (Ausubel *et al*., 1989, above). Other hybridization conditions for longer oligonucleotide molecules of the present invention can be determined by the skilled artisan using standard techniques.

Suitably designed primers can be used to detect the presence of harA-specific nucleic acids in a sample of animal tissue or fluid, including brain tissue, lung tissue, intestinal tissue, placental tissue, blood, cerebrospinal fluid, feces, mucous, urine, amniotic fluid, etc. The production of a specific amplification product can support a diagnosis of harA-mediated resistance, while lack of an amplified product can point to a lack of harA-mediated resistance. Methods for conducting amplifications, such as the polymerase chain reaction (PCR), are well known in the art. Other amplification techniques known in the art, e.g., the ligase chain reaction, can alternatively be used. The sequences of the nucleic acids disclosed herein can also be used to design primers for use in isolating homologous genes from other species or strains of *E. faecalis* or other bacterial cells.

### Recombinant Expression Systems

Recombinant vectors of the present invention, particularly expression vectors, are preferably constructed so that the coding sequence for the polynucleotide molecule of the invention is in operative association with one or more regulatory elements necessary for transcription and translation of the coding sequence to produce a polypeptide. As used herein, the term "regulatory element" includes but is not limited to nucleotide sequences that encode inducible and non-inducible promoters, enhancers, operators, ribosome-binding sites, and other elements known in the art that serve to drive and/or regulate expression of polynucleotide coding sequences. Also, as used herein, a nucleotide sequence, preferably a coding sequence or open reading frame, is "operatively associated" "operably linked" or in "operable linkage" or is in "operative association" or "operable association" with one or more regulatory elements where the regulatory elements effectively regulate and allow for the transcription of the coding sequence or the translation of its mRNA, or both.

Methods are well-known in the art for constructing recombinant vectors containing particular coding sequences in operative association with appropriate regulatory elements, and these can be used to practice the present invention. These methods include *in vitro* recombinant techniques, synthetic techniques, and *in vivo* genetic recombination. See, e.g., the techniques described in Maniatis *et al*., 1989, above; Ausubel *et al*., 1989, above; Sambrook *et al*., 1989, above; Innis *et* al., 1995, above; and Erlich, 1992, above.

A variety of expression vectors are known in the art which can be utilized to express the harA coding sequences of the present invention, including recombinant bacteriophage DNA, plasmid DNA, and cosmid DNA expression vectors containing the particular coding sequences. Typical prokaryotic expression vector plasmids that can be engineered to contain a polynucleotide molecule of the present invention include pUC8, pUC9, pBR322 and pBR329 (Biorad Laboratories, Richmond, CA), pPL and pKK223 (Pharmacia, Piscataway, NJ), pQE50 (Qiagen, Chatsworth, CA), and pGEM-T EASY (Promega, Madison, WI), among many others. Typical eukaryotic expression vectors that can be engineered to contain a nucleic acid molecule of the present invention include an ecdysone-inducible mammalian expression system (Invitrogen, Carlsbad, CA), cytomegalovirus promoter-enhancer-based systems (Promega, Madison, WI; Stratagene, La Jolla, CA; Invitrogen), and baculovirus-based expression systems (Promega), among others. Other eukaryotic vectors include pcDNA-3, pcEXV, and fusion protein vectors such as pGEX, pMal and pTrxFus. Yeast vectors which may be used include, e.g., YEp13, Yip5 and YAC vectors such as pYAC3.

The regulatory elements of these and other vectors can vary in their strength and specificities. Depending on the host/vector system utilized, any of a number of suitable transcription and translation elements can be used. For instance, when cloning in mammalian cell systems, promoters isolated from the genome of mammalian cells, e.g., mouse metallothionein promoter, or from viruses that grow in these cells, e.g., vaccinia virus 7.5K promoter or Moloney murine sarcoma virus long terminal repeat, can be used. Promoters obtained by recombinant DNA or synthetic techniques can also be used to provide for transcription of the inserted sequence. In addition, expression from certain promoters can be elevated in the presence of particular inducers, e.g., zinc and cadmium ions for metallothionein promoters. Non-limiting examples of transcriptional regulatory regions or promoters include for bacteria, the β-gal promoter, the T7 promoter, the TAC promoter, λ left and right promoters, trp and lac promoters, trp-lac fusion promoters, etc.; for yeast, glycolytic enzyme promoters, such as ADH-I and -II promoters, GPK promoter, PGI promoter, TRP promoter, *etc.;* and for mammalian cells, SV40 early and late promoters, adenovirus major late promoters, among others. The present invention further provides a polynucleotide molecule comprising the nucleotide sequence of the promoter of the harA gene of E. faecalis, contained in plasmid pGEM-T/harA, which can be used to express any of the coding sequences of the present invention in E. coli or other suitable hosts.

Specific initiation signals are also required for efficient translation of inserted coding sequences. These signals typically include an ATG initiation codon and adjacent sequences. In cases where the polynucleotide molecule of the present invention including its own initiation codon and adjacent sequences are inserted into the appropriate expression vector, no additional translation control signals may be needed. However, in cases where only a portion of a coding sequence is inserted, exogenous translational control signals, including the ATG initiation codon, may be required. These exogenous translational control signals and initiation codons can be obtained from a variety of sources, both natural and synthetic. Furthermore, the initiation codon must be in phase with the reading frame of the coding regions to ensure in-frame translation of the entire insert.

Expression vectors can also be constructed that will express a fusion polypeptide comprising a polypeptide or polypeptide of the present invention. Such fusion polypeptides can be used, *e*.*g*., to raise antisera against an E. faecalis harA polypeptide, to study the biochemical properties of the E. faecalis harA polypeptide, to engineer an E. faecalis harA polypeptide exhibiting different immunological or functional properties, or to aid in the identification or purification, or to improve the stability, of a recombinantly-expressed E. faecalis harA polypeptide. Possible fusion polypeptide expression vectors include but are not limited to vectors incorporating sequences that encode β-galactosidase and trpE fusions, maltose-binding polypeptide fusions (pMal series; New England Biolabs), glutathione-S-transferase fusions (pGEX series; Pharmacia), polyhistidine fusions (pET series; Novagen Inc., Madison, WI), and thioredoxin fusions (pTrxFus; Invitrogen, Carlsbad, CA) and the FLAG epitope fusion partner (see below). Methods are well-known in the art for constructing expression vectors encoding these and other fusion polypeptides.

The fusion polypeptide can be useful to aid in purification of the expressed polypeptide. In non-limiting embodiments, *e.g.*, a harA-maltose-binding fusion polypeptide can be purified using amylose resin; a HtaA -glutathione-S-transferase fusion polypeptide can be purified using glutathione-agarose beads; and a harA -polyhistidine fusion polypeptide can be purified using divalent nickel resin. Alternatively, antibodies against a carrier polypeptide or peptide can be used for affinity chromatography purification of the fusion polypeptide. For example, a nucleotide sequence coding for the target epitope of a monoclonal antibody can be engineered into the expression vector in operative association with the regulatory elements and situated so that the expressed epitope is fused to a E. faecalis polypeptide of the present invention. In a non-limiting embodiment, a nucleotide sequence coding for the FLAG™ epitope tag (International Biotechnologies Inc.), which is a hydrophilic marker peptide, can be inserted by standard techniques into the expression vector at a point corresponding to the amino or carboxyl terminus of the harA polypeptide. The expressed harA polypeptide-FLAG™ epitope fusion product can then be detected and affinity-purified using commercially available anti-FLAG™ antibodies.

The expression vector can also be engineered to contain polylinker sequences that encode specific protease cleavage sites so that the expressed E. *faecalis* polypeptide can be released from the carrier region or fusion partner by treatment with a specific protease. For example, the fusion polypeptide vector can include a nucleotide sequence encoding a thrombin or factor Xa cleavage site, among others.

A signal sequence upstream from and in the same reading frame with the *E. faecalis* coding sequence can be engineered into the expression vector by known methods to direct the trafficking and secretion of the expressed polypeptide. Non-limiting examples of signal sequences include those from α-factor, immunoglobulins, outer membrane polypeptides, penicillinase, and T-cell receptors, among others.

To aid in the selection of host cells transformed or transfected with a recombinant vector of the present invention, the vector can be engineered to further comprise a coding sequence for a reporter gene product or other selectable marker. Such a coding sequence is preferably in operative association with the regulatory elements, as described above. Reporter genes that are useful in practicing the invention are well-known in the art and include those encoding chloramphenicol acetyltransferase (CAT), green fluorescent polypeptide, firefly luciferase, and human growth hormone, among others. Nucleotide sequences encoding selectable markers are well-known in the art, and include those that encode gene products conferring resistance to antibiotics or anti-metabolites, or that supply an auxotrophic requirement. Examples of such sequences include those that encode thymidine kinase activity, or resistance to methotrexate, ampicillin, kanamycin, chloramphenicol, zeocin, pyrimethamine, aminoglycosides, or hygromycin, among others.

The present invention further provides transformed host cells comprising a polynucleotide molecule or recombinant vector of the present invention, and cell lines derived therefrom. Host cells useful in practicing the invention can be eukaryotic or prokaryotic cells. Such transformed host cells include but are not limited to microorganisms, such as bacteria transformed with recombinant bacteriophage DNA, plasmid DNA or cosmid DNA vectors, or yeast transformed with a recombinant vector, or animal cells, such as insect cells infected with a recombinant virus vector, *e.g.*, baculovirus, or mammalian cells infected with a recombinant virus vector, *e*.*g*., adenovirus or vaccinia virus, among others. For example, a strain of *E. coli* can be used, such as, *e.g.,* the DH5α strain available from the ATCC, Rockville, MD, USA (Accession No. 31343), or from GIBCO BRL, Gaithersburg, MD. Anther example of a suitable host is *Staphylococcus aureus.* Eukaryotic host cells include yeast cells, although mammalian cells, *e.g.,* from a mouse, hamster, cow, monkey, or human cell line, among others, can also be utilized effectively. Examples of eukaryotic host cells that can be used to express a recombinant polypeptide of the invention include Chinese hamster ovary (CHO) cells (*e.g.*, ATCC Accession No. CCL-61), NIH Swiss mouse embryo cells NIH/3T3 (*e.g*., ATCC Accession No. CRL-1658), and Madin-Darby bovine kidney (MDBK) cells (ATCC Accession No. CCL-22), as well as Sf-9 cells. Transfected cells can express the nucleic acids of the invention by chromosomal integration, or episomally.

The recombinant vector of the invention is preferably transformed or transfected into one or more host cells of a substantially homogeneous culture of cells. The vector is generally introduced into host cells in accordance with known techniques, such as, *e.g.*, by protoplast transformation, calcium phosphate precipitation, calcium chloride treatment, microinjection, electroporation, transfection by contact with a recombined virus, liposome-mediated transfection, DEAE-dextran transfection, transduction, conjugation, or microprojectile bombardment, among others. Selection of transformants can be conducted by standard procedures, such as by selecting for cells expressing a selectable marker, e.g., antibiotic resistance, associated with the recombinant expression vector.

Once an expression vector is introduced into the host cell, the integration and maintenance of the polynucleotide molecule of the present invention, either in the host cell genome or episomally, can be confirmed by standard techniques, *e.g.*, by Southern hybridization analysis, restriction enzyme analysis, PCR analysis including reverse transcriptase PCR (rt-PCR), or by immunological assay to detect the expected polypeptide product. Host cells containing and/or expressing a polynucleotide molecule of the present invention can be identified by any of at least four general approaches that are well-known in the art, including: (i) DNA-DNA, DNA-RNA, or RNA-antisense RNA hybridization; (ii) detecting the presence of "marker" gene functions; (iii) assessing the level of transcription as measured by the expression of specific mRNA transcripts in the host cell; or (iv) detecting the presence of mature polypeptide product, *e.g.*, by immunoassay, as known in the art.

### Expression And Purification Of Recombinant Polypeptides

Once a nucleic acid of the present invention has been stably introduced into an appropriate host cell, the transformed host cell is clonally propagated, and the resulting cells are grown under conditions conducive to the maximum production of the encoded polypeptide. Such conditions typically include growing transformed cells to high density. Where the expression vector comprises an inducible promoter, appropriate induction conditions such as, *e.g.*, temperature shift, exhaustion of nutrients, addition of gratuitous inducers (*e*.*g*., analogs of carbohydrates, such as isopropyl-β-D-thiogalactopyranoside (IPTG)), accumulation of excess metabolic by-products, or the like, are employed as needed to induce expression.

Where the polypeptide is retained inside the host cells, the cells are harvested and lysed, and the product is substantially purified or isolated from the lysate under extraction conditions known in the art to minimize polypeptide degradation such as, *e*.*g*., at 4°C, or in the presence of protease inhibitors, or both. Where the polypeptide is secreted from the host cells, the exhausted nutrient medium can simply be collected and the polypeptide substantially purified or isolated therefrom.

The polypeptide can be substantially purified or isolated from cell lysates or culture medium, as necessary, using standard methods, including but not limited to one or more of the following methods: ammonium sulfate precipitation, size fractionation, ion exchange chromatography, HPLC, density centrifugation, and affinity chromatography. If the polypeptide lacks biological activity, it can be detected as based, *e.g.*, on size, or reactivity with a polypeptide-specific antibody, or by the presence of a fusion tag. For use in practicing the present invention, the polypeptide can be in an unpurified state as secreted into the culture fluid or as present in a cell lysate, but is preferably substantially purified or isolated therefrom. In an embodiment of the invention, the polypeptide is present in a preparation in at least about a 1000x higher concentration than its natural counterpart is normally found in a preparation of *E. faecalis* cell lysate.

### Polypeptides

The present invention provides a method of preparing any of the polypeptides described above, comprising culturing a host cell transformed with a recombinant expression vector, said recombinant expression vector comprising a polynucleotide molecule comprising a nucleotide sequence encoding the particular polypeptide, which polynucleotide molecule is in operative association with one or more regulatory elements, under conditions conducive to the expression of the polypeptide, and recovering the expressed polypeptide from the cell culture.

Once a polypeptide of the present invention of sufficient purity has been obtained, it can be characterized by standard methods, including by SDS-PAGE, size exclusion chromatography, amino acid sequence analysis, immunological activity, biological activity, etc. The polypeptide can be further characterized using hydrophilicity analysis (see, *e.g*., Hopp and Woods, 1981, Proc. Natl. Acad. Sci. *USA* 78:3824), or analogous software algorithms, to identify hydrophobic and hydrophilic regions. Structural analysis can be carried out to identify regions of the polypeptide that assume specific secondary structures. Biophysical methods such as X-ray crystallography (Engstrom, 1974, Biochem. Exp. Biol. 11: 7-13), computer modeling (Fletterick and Zoller (eds), 1986, in: Current Communications in Molecular Biology, Cold Spring Harbor Laboratory, Cold Spring Harbor, NY), and nuclear magnetic resonance (NMR) can be used to map and study potential sites of interaction between the polypeptide and other putative interacting polypeptides/receptors/molecules. Information obtained from these studies can be used to design deletion mutants and vaccine compositions, and to design or select therapeutic or pharmacologic compounds that can specifically block the biological function of the polypeptide *in vivo.*

Polypeptides of the present invention are useful for a variety of purposes, including as diagnostic reagents, *e*.*g*., using standard techniques such as ELISA assays, to screen for *E. faecalis*-specific antibodies in blood or serum samples from animals; or as antigens to raise polyclonal or monoclonal antibodies, as described below, which antibodies are useful as diagnostic reagents, *e.g.*, using standard techniques such as Western blot assays, to screen for *E. faecalis*-specific polypeptides in cell, tissue or fluid samples from an animal.

Polypeptides of the present invention are especially useful in the screening of compounds to identify modulators of harA activity, e.g. in the treatment of Enterococcal infections as well as staphylococcal and pneumococcal infections.

Methods for determining binding of compounds to polypeptides are well known in the art, and include assays to measure binding directly, or competitive binding assays. Such binding assays may utilize a compound labeled so as to facilitate detection of the compound, e.g., radiolabeling the compound using ³H, ¹⁴C, ³²P, or by chemical conversion of the compound bound to the polypeptide, or using ELISA or other methods involving immobilization of either the compound or the polypeptide.

### Analogs And Derivatives Of Polypeptides

One or more chemical modifications of the polypeptide can be carried out using known techniques to prepare analogs therefrom, including but not limited to any of the following: substitution of one or more L-amino acids of the polypeptide with corresponding D-amino acids, amino acid analogs, or amino acid mimics, so as to produce, *e.g.,* carbazates or tertiary centers; or specific chemical modification, such as, *e.g.*, proteolytic cleavage with trypsin, chymotrypsin, papain or V8 protease, or treatment with NaBH₄ or cyanogen bromide, or acetylation, formylation, oxidation or reduction, *etc.* Alternatively or additionally, polypeptides of the present invention can be modified by genetic recombination techniques.

A polypeptide of the present invention can be derivatized by conjugation thereto of one or more chemical groups, including but not limited to acetyl groups, sulfur bridging groups, glycosyl groups, lipids, and phosphates, and/or by conjugation to a second polypeptide of the present invention, or to another polypeptide, such as, *e.g.,* serum albumin, keyhole limpet hemocyanin, or commercially activated BSA, or to a polyamino acid (*e*.*g*., polylysine), or to a polysaccharide, *(e.g.,* sepharose, agarose, or modified or unmodified celluloses), among others. Such conjugation is preferably by covalent linkage at amino acid side chains and/or at the N-terminus or C-terminus of the polypeptide. Methods for carrying out such conjugation reactions are well-known in the field of polypeptide chemistry.

Derivatives useful in practicing the claimed invention also include those in which a water-soluble polymer such as, *e.g.,* polyethylene glycol, is conjugated to a polypeptide of the present invention, or to an analog or derivative thereof, thereby providing additional desirable properties while retaining, at least in part, the immunogenicity of the polypeptide. These additional desirable properties include, *e.g.,* increased solubility in aqueous solutions, increased stability in storage, increased resistance to proteolytic dehydration, and increased *in vivo* half-life. Water-soluble polymers suitable for conjugation to a polypeptide of the present invention include but are not limited to polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, and α,β-poly[2-hydroxyethyl]-DL-aspartamide. Polyethylene glycol is particularly preferred. Methods for making water-soluble polymer conjugates of polypeptides are known in the art and are described in, among other places, U.S. Patent 3,788,948; U.S. Patent 3,960,830; U.S. Patent 4,002,531; U.S. Patent 4,055,635; U.S. Patent 4,179,337; U.S. Patent 4,261,973; U.S. Patent 4,412,989; U.S. Patent 4,414,147; U.S. Patent 4,415,665; U.S. Patent 4,609,546; U.S. Patent 4,732,863; U.S. Patent 4,745,180; European Patent (EP) 152,847; EP 98,110; and Japanese Patent 5,792,435, which patents are incorporated herein by reference.

### Antibodies

The present invention further provides isolated antibodies directed against a polypeptide of the present invention. In a preferred embodiment, antibodies can be raised against a harA polypeptide from E. faecalis or B. subtilis using known methods. Various host animals selected from pigs, cows, horses, rabbits, goats, sheep, rats or mice, can be immunized with a partially or substantially purified, or isolated, E. faecalis polypeptide, or with a homologue, fusion polypeptide, substantial portion, analog or derivative thereof, as these are described above. An adjuvant, such as described below, can be used to enhance antibody production.

Polyclonal antibodies can be obtained and isolated from the serum of an immunized animal and tested for specificity against the antigen using standard techniques. Alternatively, monoclonal antibodies can be prepared and isolated using any technique that provides for the production of antibody molecules by continuous cell lines in culture. These include but are not limited to the hybridoma technique originally described by Kohler and Milstein (Nature, 1975, 256: 495-497); the human B-cell hybridoma technique (Kosbor *et al.,* 1983, Immunology Today 4:72; Cote *et al.,* 1983, Proc. Natl. Acad. Sci. *USA* 80: 2026-2030); and the EBV-hybridoma technique (Cole *et al*., 1985, Monoclonal Antibodies and Cancer Therapy, Alan R. Liss, Inc., pp. 77-96). Alternatively, techniques described for the production of single chain antibodies (see, *e.g*., U.S. Patent 4,946,778) can be adapted to produce harA antigen-specific single chain antibodies. These publications are incorporated herein by reference.

The production of humanized antibodies is now well-known in the art. One manner in which humanized antibodies may be produced is by transfer of the complementarity determining regions of the antibody to a human monoclonal antibody, for example, as described in Jones, P. et al. Nature 321:52-525 (1986) or Tempest et al., Biotechnology 9:266-273 (1991). Humanized antibodies may be made also by transgenic mice engineered to express human heavy and light chains in response to an antigen, for example as described in J. Immunol. Meth. 231:11-23 (1999).

Antibody fragments that contain specific binding sites for a polypeptide of the present invention are also encompassed within the present invention, and can be generated by known techniques. Such fragments include but are not limited to F(ab')₂ fragments, which can be generated by pepsin digestion of an intact antibody molecule, and Fab fragments, which can be generated by reducing the disulfide bridges of the F(ab')₂ fragments. Alternatively, Fab expression libraries can be constructed (Huse *et al.,* 1989, Science 246: 1275-1281) to allow rapid identification of Fab fragments having the desired specificity to the *E. faecalis* polypeptide.

Techniques for the production and isolation of monoclonal antibodies and antibody fragments are well-known in the art, and are additionally described, among other places, in Harlow and Lane, 1988, Antibodies: A Laboratory Manual, Cold Spring Harbor Laboratory, and in J. W. Goding, 1986, Monoclonal Antibodies: Principles and Practice, Academic Press, London, which are incorporated herein by reference.

### Targeted Mutation Of E. faecalis Genes

Based on the disclosure of the nucleic acids of the present invention, genetic constructs can be prepared for use in disabling or otherwise mutating a harA gene (which gene is hereinafter referred to as the "harA gene"). The harA gene can be mutated using an appropriately designed genetic construct. For example, the harA gene can be mutated using a genetic construct of the present invention that functions to: (a) delete all or a portion of the coding sequence or regulatory sequence of the harA gene; or (b) replace all or a portion of the coding sequence or regulatory sequence of the harA gene with a different nucleotide sequence; or (c) insert into the coding sequence or regulatory sequence of the harA gene one or more nucleotides, or an oligonucleotide molecule, or polynucleotide molecule, which can comprise a nucleotide sequence from harA or from a heterologous source; or (d) carry out some combination of (a), (b) and (c). Alternately, constructs can be employed to alter the expression of the harA gene or the stability of its encoded polypeptide.

Cells in which the harA gene has been mutated are, for example, useful in practicing the present invention, e.g., where such cells carrying the disabled gene can be used in a vaccine composition, particularly in a modified live vaccine, to induce or contribute to the induction of, a protective response in an animal. The invention also encompasses cells expressing polypeptides and polypeptides of the invention where such cells are constitutive mutants.

In a non-limiting embodiment, a genetic construct of the present invention is used to mutate a wild-type harA gene by replacement of the coding sequence of the wild-type gene, or a promoter or other regulatory region thereof, or a portion thereof, with a different nucleotide sequence such as, e.g., a mutated coding sequence or mutated regulatory region, or portion thereof. Mutated harA gene sequences for use in such a genetic construct can be produced by any of a variety of known methods, including by use of error-prone PCR, or by cassette mutagenesis. For example, oligonucleotide-directed mutagenesis can be employed to alter the coding sequence or promoter sequence of a wild-type harA gene in a defined way, e.g., to introduce a frame-shift or a termination codon at a specific point within the sequence. Alternatively or additionally, a mutated nucleotide sequence for use in the genetic construct of the present invention can be prepared by insertion or deletion of the coding sequence or promoter sequence of one or more nucleotides, oligonucleotide molecules or polynucleotide molecules, or by replacement of a portion of the coding sequence or promoter sequence with one or more different nucleotides, oligonucleotide molecules or polynucleotide molecules. Such oligonucleotide molecules or polynucleotide molecules can be obtained from any naturally occurring source or can be synthetic. The inserted or deleted sequence can serve simply to disrupt the reading frame of the harA gene, or can further encode a heterologous gene product such as a selectable marker.

Alternatively or additionally, random mutagenesis can be used to produce a mutated harA gene sequence for use in a genetic construct of the present invention. Random mutagenesis can be carried out by any suitable techniques such as, e.g., by exposing cells carrying a harA gene to ultraviolet radiation or x-rays, or to chemical mutagens such as N-methyl-N'-nitrosoguanidine, ethyl methane sulfonate, nitrous acid or nitrogen mustards, and then selecting for cells carrying a mutation in the particular gene. See, e.g., Ausubel, 1989, above, for a review of mutagenesis techniques.

Mutations to produce modified harA polypeptides that are useful in practicing the present invention can occur anywhere in the harA gene, including in the ORF, or in the promoter or other regulatory region, or in any other sequences that naturally comprise the gene or ORF, or that alter expression of the gene or the stability of its encoded polypeptide.

Alternatively, a genetic construct of the present invention can comprise nucleotide sequences that naturally flank the harA gene or ORF *in situ,* with only a portion or no nucleotide sequences from the coding region of the gene itself. Such a genetic construct would be useful, e.g., to delete the entire harA gene or ORF.

For targeted gene mutation through homologous recombination, the genetic construct is preferably a plasmid, either circular or linearized, comprising a mutated nucleotide sequence as described above. In a non-limiting embodiment, at least about 200 nucleotides of the mutated sequence are used to specifically direct the genetic construct of the present invention to the harA gene for homologous recombination, although shorter lengths of nucleotides can also be effective. In addition, the plasmid preferably comprises an additional nucleotide sequence encoding a reporter gene product or other selectable marker that is constructed so that it will insert into the harA gene in operative association with the regulatory element sequences of the native harA gene to be disrupted. Reporter genes that can be used in practicing the invention are well-known in the art and include those encoding CAT, green fluorescent polypeptide, and β-galactosidase, among others. Nucleotide sequences encoding selectable markers are also well-known in the art, and include those that encode gene products conferring resistance to antibiotics or anti-metabolites, or that supply an auxotrophic requirement. Examples of such sequences include those that encode pyrimethamine resistance, or neomycin phosphotransferase (which confers resistance to aminoglycosides).

Methods that can be used for creating the genetic constructs of the present invention are well-known in the art, and include *in vitro* recombinant techniques, synthetic techniques, and *in vivo* genetic recombination, as described, among other places, in Maniatis *et al.,* 1989, above; Ausubel *et al*., 1989, above; Sambrook *et al*., 1989, above; Innis *et al*., 1995, above; and Erlich, 1992, above.

Cells can be transformed or transfected with a genetic construct of the present invention in accordance with known techniques, such as, *e.g*., by electroporation. Selection of transformants can be carried out using standard techniques, such as by selecting for cells expressing a selectable marker associated with the construct. Identification of transformants in which a successful recombination event has occurred and the particular target gene has been altered can be carried out by genetic analysis, such as by Southern blot analysis, or by Northern analysis to detect a lack of mRNA transcripts encoding the particular polypeptide, or cells lacking the particular polypeptide, as determined, *e.g.*, by immunological analysis, by the appearance of a novel phenotype, such as reduced pathogenicity, by PCR assay, or by some combination thereof.

In a further non-limiting embodiment, the genetic construct of the present invention can additionally comprise a different gene or coding region from E. faecalis or from a different pathogen that infects the animal, which gene or coding region encodes an antigen useful to induce, or contribute to the induction of, a separate and distinct protective immune response in the animal upon vaccination with the modified live cells of the present invention. This additional gene or coding region can be further engineered to contain a signal sequence that leads to secretion of the encoded antigen from the modified live cell, thereby allowing for the antigen to be displayed to the immune system of the vaccinated animal.

The present invention thus provides modified live *E. faecalis* cells in which the harA gene has been mutated. In addition, the present invention provides a method of preparing modified live cells expressing harA, comprising: (a) transforming cells with a genetic construct of the invention; (b) selecting transformed cells in which the harA gene has been mutated by the genetic construct; and (c) selecting from among the cells of step (b) those cells that can be used in a vaccine to protect a susceptible animal. The invention also encompasses killed cell compositions prepared from such modified cells.

The invention also relates to a kit for detecting the presence of a harA-specific amino acid or nucleotide sequence, or an anti-harA antibody. The kit can also contain means for detecting the polypeptide, polynucleotide, or antibody of the invention including, for example, an enzyme, fluorescent, or radioactive label attached to the polypeptide, polynucleotide, or antibody, or attached to a moiety that binds to the polypeptide, polynucleotide, or antibody.

The nucleic acids of the invention comprising probes for hybridization, or equivalently, for PCR amplification of target sequences, e.g., in an organism containing or expressing a harA gene, preferably comprise isolated DNA comprising a fragment of 15-50 nucleotides. Such harA-specific nucleic acids can be detected using hybridization assays such as are well-known in the art and described below in exemplary manner.

By way of example and not limitation, conditions of high stringency comprise, for example: prehybridization of filters containing DNA, carried out for 8 h to overnight at 65°C in buffer composed of 6X SSC, 50 mM Tris-HCI (pH 7.5), 1 mM EDTA, 0.02% PVP, 0.02% Ficoll, 0.02% BSA, and 500 µg/mL denatured salmon sperm DNA, followed by hybridization for 48 h at 65°C in the prehybridization mixture, wherein the mixture contains now 100 µg/mL denatured salmon sperm DNA and 5-20 X 10⁶ cpm of ³²P-labeled probe, followed by washing of filters at 37°C for 1 h in a solution containing 2X SSC, 0.01% PVP, 0.01% Ficoll, and 0.01% BSA. This is followed by a wash in 0.1X SSC at 50°C for 45 min before autoradiography.

Other conditions of high stringency which may be used depend on the nature of the nucleic acid (*e.g*. length, GC content, *etc*.) and the purpose of the hybridization (detection, amplification, *etc.)* and are well known in the art. For example, stringent hybridization of an oligonucleotide of approximately 15-40 bases to a complementary sequence in the polymerase chain reaction (PCR) is done under the following conditions: a salt concentration of 50 mM KCI, a buffer concentration of 10 mM Tris-HCI, a Mg²⁺ concentration of 1.5 mM, a pH of 7-7.5 and an annealing temperature of 55-60°C. Hybridization and wash conditions are well known and exemplified in Sambrook, et al., Molecular Cloning: A Laboratory Manual, Second Edition, Cold Spring Harbor, N.Y., (1989), particularly Chapter 11 therein.

In another specific embodiment, a nucleic acid which is hybridizable to a harA nucleic acid, or its complement, under conditions of moderate stringency is provided. Selection of appropriate conditions for such stringencies is well known in the art (*see e.g.*, Sambrook et al., 1989, Molecular Cloning, A Laboratory Manual, 2d Ed., Cold Spring Harbor Laboratory Press, Cold Spring Harbor, New York; *see also,* Ausubel et al., eds., in the Current Protocols in Molecular Biology series of laboratory technique manuals, © 1987-1997, Current Protocols, © 1994-1997 John Wiley and Sons, Inc.). For medium stringency hybridization, membranes are additionally subjected to four washes each for 30 minutes in 40 mM sodium phosphate, pH 7.2, 1% SDS, 1 mM EDTA at 55°C.

By way of example and not limitation, procedures using such conditions of low stringency for regions of hybridization of over 90 nucleotides are as follows (*see also* Shilo and Weinberg, 1981, Proc. Natl. Acad. Sci. U.S.A. 78, 6789-6792). Filters containing DNA are pretreated for 6 h at 40°C in a solution containing 35% formamide, 5X SSC, 50 mM Tris-HCI (pH 7.5), 5 mM EDTA, 0.1% PVP, 0.1% Ficoll, 1% BSA, and 500 µg/mL denatured salmon sperm DNA. Hybridizations are carried out in the same solution with the following modifications: 0.02% PVP, 0.02% Ficoll, 0.2% BSA, 100 µg/mL salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCI (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency which may be used are well known in the art (*e.g.*, as employed for cross-species hybridizations).

## Claims

1. A method of screening a compound to determine whether the compound binds to a harA polypeptide, which comprises: contacting the harA polypeptide with the compound under conditions suitable for binding; and detecting whether the compound binds to the harA polypeptide, wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4).

2. A method of screening a compound to determine whether the compound inhibits harA activity, which comprises: measuring the activity of a harA polypeptide (i) in the absence and (ii) in the presence of the compound, under conditions suitable for measuring harA activity; wherein a decrease in harA activity in the presence of the compound compared to the harA activity in the absence of the compound identifies the compound as inhibiting harA activity, wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4).

3. The method of claim 2, wherein the harA activity measured is NTPase activity.

4. The use of a hygromycin A-resistant strain of E. faecalis or B. subtilis to determine whether an antibacterial agent is effective in treating organisms which exhibit harA-mediated drug resistance.

5. The use of a nucleic acid encoding a harA polypeptide to identify an organism containing a harA gene, wherein the nucleic acid comprises a nucleotide sequence selected from the group consisting of:
(a) a nucleotide sequence which is at least 75% identical to (i) the nucleotide sequence shown in Figure 1 (SEQ ID NO:1) or (ii) the nucleotide sequence shown in Figure 3 (SEQ ID NO:3);
(b) a nucleotide sequence complementary to the nucleotide sequence of (a); and
(c) a nucleotide sequence that is degenerate to the nucleotide sequence of (a) or (b).

6. A recombinant vector comprising a nucleic acid encoding a harA polypeptide, wherein the nucleic acid comprises a nucleotide sequence selected from the group consisting of:
(a) a nucleotide sequence which is at least 75% identical to (i) the nucleotide sequence shown in Figure 1 (SEQ ID NO:1) or (ii) the nucleotide sequence shown in Figure 3 (SEQ ID NO:3);
(b) a nucleotide sequence complementary to the nucleotide sequence of (a); and
(c) a nucleotide sequence that is degenerate to the nucleotide sequence of (a) or (b).

7. A vector of claim 6 selected from pMP, pJPM1, pGEM-T, pVA891, pSK⁻, pUC8, pUC9, pBR322, pBR329, pPL, pKK223 and pQE50.

8. A vector of claim 7 which is pMP-bac-A1-1 (ATCC Accession No. PTA-2551) or pGEM-T/harA (ATCC Accession No. PTA-2552).

9. A recombinant vector comprising nucleic acid encoding a harA polypeptide, wherein the nucleic acid encoding the harA polypeptide comprises a nucleotide sequence comprising a chloramphenicol resistance insertion or an erythromycin resistance insertion.

10. A cell comprising the vector of any one of claims 6 - 9.

11. The cell of claim 10 which is the Bacillus subtilis strain JH642 expZ::CAT (ATCC Accession No. PTA-2480) or the Enterococcus faecalis strain OG1X harA::ERM (ATCC Accession No. PTA-2550).

12. A recombinant harA polypeptide comprising an amino acid sequence which is at least 75% identical to (i) the amino acid sequence shown in Figure 2 (SEQ ID NO:2) or (ii) the amino acid sequence shown in Figure 4 (SEQ ID NO:4).

13. An immunological composition comprising the harA polypeptide of claim 12 and a pharmaceutically acceptable carrier.

14. An immunological composition of claim 13, which further comprises an adjuvant.

15. A pharmaceutical composition comprising a harA polypeptide and a pharmaceutically acceptable carrier, wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4).

16. A pharmaceutical composition comprising a nucleic acid encoding a harA polypeptide and a pharmaceutically acceptable carrier wherein the nucleic acid comprises a nucleotide sequence selected from the group consisting of:
(a) a nucleotide sequence which is at least 75% identical to (i) the nucleotide sequence shown in Figure 1 (SEQ ID NO:1) or (ii) the nucleotide sequence shown in Figure 3 (SEQ ID NO:3);
(b) a nucleotide sequence complementary to the nucleotide sequence of (a); and
(c) a nucleotide sequence that is degenerate to the nucleotide sequence of (a) or (b).

17. A harA polypeptide for use as a medicament wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4).

18. A nucleic acid encoding a harA polypeptide for use as a medicament, wherein the nucleic acid comprises a nucleotide sequence selected from the group consisting of:
(a) a nucleotide sequence which is at least 75% identical to (i) the nucleotide sequence shown in Figure 1 (SEQ ID NO:1) or (ii) the nucleotide sequence shown in Figure 3 (SEQ ID NO:3);
(b) a nucleotide sequence complementary to the nucleotide sequence of (a); and
(c) a nucleotide sequence that is degenerate to the nucleotide sequence of (a) or (b).

19. The use of a harA polypeptide in the manufacture of a medicament for the treatment of a drug resistant infection wherein the harA polypeptide comprises an amino acid sequence at least 75% identical to (a) the amino acid sequence shown in Figure 2 (SEQ ID NO:2) or (b) the amino acid sequence shown in Figure 4 (SEQ ID NO:4).

20. The use of a nucleic acid encoding a harA polypeptide in the manufacture of a medicament for the treatment of a drug resistant infection, wherein the nucleic acid comprises a nucleotide sequence selected from the group consisting of:
(a) a nucleotide sequence which is at least 75% identical to (i) the nucleotide sequence shown in Figure 1 (SEQ ID NO:1) or (ii) the nucleotide sequence shown in Figure 3 (SEQ ID NO:3);
(b) a nucleotide sequence complementary to the nucleotide sequence of (a); and
(c) a nucleotide sequence that is degenerate to the nucleotide sequence of (a) or (b).
